# EUROPEAN PATENT SPECIFICATION

(11) **EP 3 761 961 B1**
(45) Date of publication and mention of the grant of the patent: **02.12.2026**
(21) Application number: 19764228.3
(22) Date of filing: 06.03.2019
(51) Int. Cl.: A61K 9/08, A61K 47/14, A61K 47/54, A61K 9/00, A61K 31/506, A61K 31/5513, A61K 47/24, A61K 47/28

(54) **AQUEOUS FORMULATIONS FOR INSOLUBLE DRUGS**
WÄSSRIGE FORMULIERUNGEN FÜR UNLÖSLICHE ARZNEIMITTEL
FORMULATIONS AQUEUSES POUR MÉDICAMENTS INSOLUBLES

(30) Priority: 07.03.2018 US 201862639710 P
(43) Date of publication of application: 13.01.2021
(73) Proprietor: Chen, Andrew Xian, San Diego, California 92131 (US)
(72) Inventor: CHU, Jan-Jon, San Diego, California 92131 (US); XU, Jianmin, San Diego, California 92131 (US); ISLAM, Mohammad Saiful, San Diego, California 92131 (US); CHEN, Andrew Xian, San Diego, California 92131 (US)
(74) Representative: Moré, Solveig Helga
(86) International application number: PCT/US2019/021029
(87) International publication number: WO 2019/173526

(56) References cited:
- EP-A2- 0 391 369
- WO-A2-2009/046444
- US-A- 4 649 155
- US-A1- 2002 107 291
- US-A1- 2002 107 291
- US-A1- 2004 052 845
- US-A1- 2015 224 121
- US-A1- 2015 272 943
- US-B1- 6 193 985
- US-B1- 6 586 407
- WEIHUI CAI ET AL: "A propofol microemulsion with low free propofol in the aqueous phase: Formulation, physicochemical characterization, stability and pharmacokinetics", INTERNATIONAL JOURNAL OF PHARMACEUTICS, vol. 436, no. 1-2, 1 October 2012 (2012-10-01), pages 536 - 544, XP055208265, ISSN: 0378-5173, DOI: 10.1016/j.ijpharm.2012.07.008

## Description

### FIELD OF THE INVENTION

This invention relates to clear and one-phase aqueous solution compositions comprising lecithin, medium chain triglycerides, a bile salt and water having utility as formulation vehicles to dissolve drugs for pharmaceutical applications as described in the appended claims.

### BACKGROUND OF THE INVENTION

Insoluble molecules account for about 40-60% of drug substances now being discovered or commercially developed. To be suitable for injection as well as many other routes of administration, these drugs must first be dissolved in a liquid vehicle. For safety reasons, an aqueous vehicle is preferred over a non-aqueous vehicle.

Various drug solubilizers have been developed to dissolve insoluble drugs, including those that use lecithin, oils, bile salts or cyclodextrins. All these prior art solubilizers have one or more of the following drawbacks, namely:
1. Not a clear or one-phase solution,
2. Unable to dissolve many drugs to be considered of general utility,
3. Require high energy to dissolve the drug,
4. Drug precipitates when diluted with water or an IV infusion fluid, and
5. Poor safety when injected into human or animals.

Several approaches have been described in the prior art for the solubilization and parenteral delivery of hydrophobic drugs. For example, US 4,649,155 A discloses injectable micellar solutions comprising a phospholipid and a cholanic acid for solubilizing vitamin E and other actives. US 2002/0107291 A1 teaches clear, injectable propofol formulations based on lecithin and bile salt systems. Cai et al. ("A propofol microemulsion with low free propofol in the aqueous phase: formulation, physicochemical characterization, stability and pharmacokinetics." International journal of pharmaceutics 436.1-2 (2012): 536-544), describe the development and optimization of propofol microemulsions utilizing lecithin, medium-chain triglycerides, and glycocholic acid, with a focus on physicochemical and biological performance. EP 0 391 369 A2 provides stable oil-in-water emulsions for hydrophobic drugs, employing MCT oil, phospholipids, non-ionic surfactants, and bile acids or salts. These disclosures illustrate the use of lecithin, MCT, and bile salts in parenteral drug delivery systems and form the basis for further improvements in solubilizer compositions and drug formulations. The present invention relates to specific four-component solution compositions comprising Lecithin, Medium chain triglycerides (MCT), a Bile salt, and Water, which are herein referred to as L, M, B, and W, respectively, in this application. For convenience, a composition of this invention is also referred to herein as an LMBW formulation, an LMBW composition or an LMBW.

An LMBW composition has certain physical and biological properties that are very useful for drug formulations, especially for insoluble drugs. An LMBW composition is free of the aforementioned drawbacks associated with other solubilizers.

### BRIEF SUMMARY OF THE INVENTION

This invention provides an injectable solubilizer composition, comprising:
a. a lecithin, having phosphatidylcholine content no less than 75% w/w based on the lecithin weight, at a concentration between about 6% and 72.5%;
b. a MCT having fatty acids of chain length of 6 to 12 carbons, at a concentration between >0% and 32.5%;
c. a bile salt at a concentration between 11.5% and 90%;
d. water, and
optionally alcohol, wherein the % is the weight percentage (w/w) over the total weight of the lecithin, MCT and bile salt and wherein the said composition is a clear and one-phase aqueous solution, and is substantially free of solid particles or oil droplets or other detectable aggregates or super molecular structures in the size range of >5 nm or greater, and wherein the pH of the composition is between 3 and 10.

Accordingly, this invention provides a clear and one-phase aqueous liquid composition comprising: a lecithin, MCT, bile salt, and water, in a four-component LMBW composition as claimed, their preparation and applications in drug formulations.

The LMBW composition provided in this invention is a one-phase solution and is not a multiple-phase system such as emulsion, liposome, mixed micelle dispersion and suspension.

In an aspect, the LMBW composition provided in this invention is a formulation vehicle without a drug.

In an aspect, the LMBW composition provided in this invention further contains a drug, cosmetic agent, diagnostic agent, or nutritional supplement (i.e., an active ingredient).

In an aspect, the LMBW composition provided in this invention contains an insoluble or a soluble drug.

In an aspect, the LMBW composition provided in this invention further contains an excipient including, but not limited to, an alcohol, preservative, antioxidant, bulking agent, or a mixture thereof.

This invention allows increasing the solubility of an insoluble drug using an LMBW composition as a liquid vehicle, a solubilizer or solubility enhancer.

The LMBW composition provided in this invention is between pH 3 and pH 10.

This invention also provides a method to make an LMBW composition as claimed.

In yet another aspect, this invention provides theLMBW composition of the invention for use in diagnosis, treatment or prophylaxis for a patient, comprising administration of an LMBW composition with or without a drug.

In an aspect, the LMBW compositions provided in this invention are for use in medicinal or nutrition supplement application.

In an aspect, the LMBW composition provided in this invention is used to increase solubility of a drug in water.

In an aspect, the LMBW compositions are suitable for injection or for oral, topical, inhalation, ophthalmic, otic, intranasal, intravaginal administration, or other routes of administration in a human or animal patient.

These and other aspects, objects and embodiments will become more apparent when read with the detailed description and figures which follow.

### BRIEF DESCRIPTION OF THE DRAWINGS

FIG. 1 illustrates a ternary phase diagram in an equilateral triangle plot graphically depicting concentrations (in % w/w based on the Dry Weight or combined weight of L, M and B) of the three components (L, M and B) in combinations with a fixed amount of water according to the present invention.
FIG. 2 illustrates a ternary phase diagram in an equilateral triangle plot graphically depicting concentrations (in % w/w based on the Dry Weight or combined weight of L, M and B) of the three components (L, M and B) in combinations according to the present invention.
FIG. 3 illustrates a ternary phase diagram in an equilateral triangle plot graphically depicting concentrations (in % w/w based on the Dry Weight or combined weight of L, M and B) of the three components (L, M and B) in combinations with a fixed amount of water according to the present invention.
FIG. 4 illustrates a ternary phase diagram in an equilateral triangle plot graphically depicting concentrations (in % w/w based on the Dry Weight or combined weight of L, M and B) of the three components (L, M and B) in combinations with a fixed amount of water according to the present invention.
FIG. 5 illustrates a ternary phase diagram in an equilateral triangle plot graphically depicting concentrations (in % w/w based on the Dry Weight or combined weight of L, M and B) of the three components (L, M and B) in combinations with a fixed amount of water according to the present invention.
FIG. 6 illustrates plasma concentration over time profiles of voriconazole in VFEND^{®} (denoted as R) and an LMBW formulation (F96, Example 5, denoted as T) following intravenous injection into beagle dogs at 3 mg/kg dose.

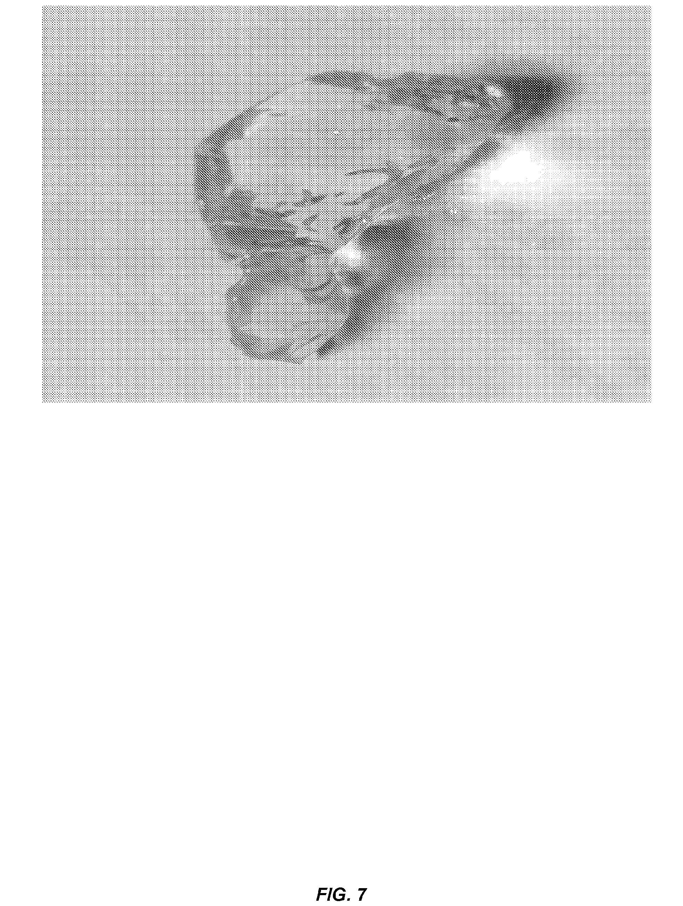

FIG. 7 illustrates a Solid LMBW (F95, Example 28), which is transparent, amorphous and glass-like.

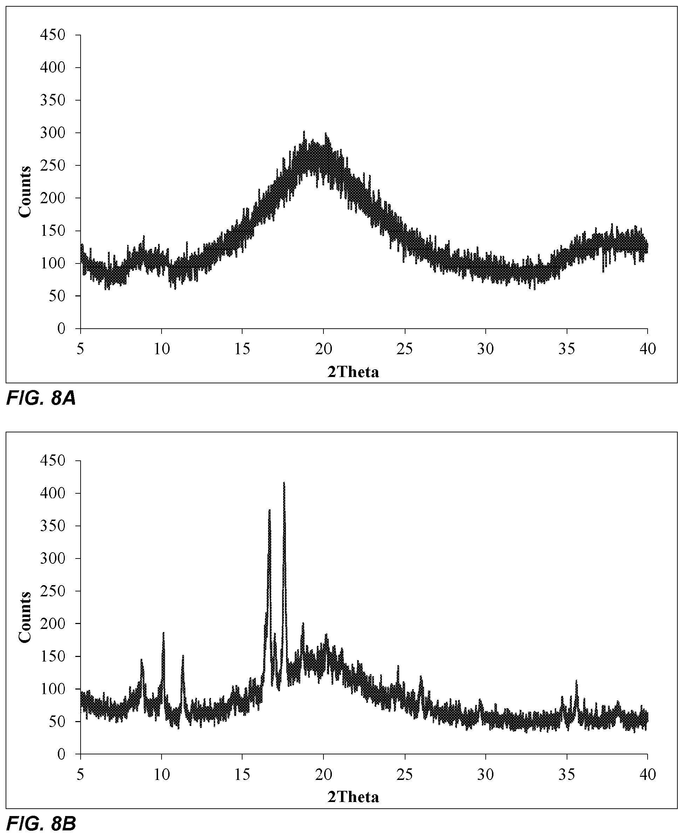

FIGS. 8A and 8B illustrate the XRPD spectrum of an amorphous Solid LMBW (F95, Example 28) in the upper panel (8A) in comparison with a crystalline Physical Mixture having the same components as the Solid LMBW but prepared by direct mixing of the components in the lower panel (8B).

### DETAILED DESCRIPTION OF THE INVENTION

### I. Definitions

The various terms used herein shall have the following definitions:
As used herein, "about" describes a quantity with a range covering 10% expansion from both sides of the target value. For example, "about 100" means any value between 90 and 110, including 90 and 110 such as 90, 91, 92, 93, 94, 95, 96, 97, 98, 99, 100, 101, 102, 103, 104, 105, 106, 107, 108, 109, and/or 110 and fractions in-between.

As used herein, an "acid" refers to any organic or inorganic acid that is suitable for pharmaceutical use. Acids that have previously been approved by the FDA for use in injectable or other solution-based drugs, or are listed on the FDA's Inactive Ingredient List, are preferred. Acids that are particularly useful for an LMBW include, but are not limited to, acetic acid, ascorbic acid, aspartic acid, benzenesulfonic, benzoic acid, citric acid, formic acid, fumaric acid, hydrochloric acid, hydrobromic acid, lactic acid, lactobionic acid, maleic acid, malic acid, malonic acid, methanesulfonic acid, phosphoric acid, propionic acid, succinic acid, sulfuric acid, and tartaric acid.

An "antioxidant" is a pharmaceutical additive that can be added to a liquid composition to prevent oxidation of the drug molecule or an inactive component. Antioxidants include, but are not limited to, reducing agents, metal ion chelating agents and inert gases. The preferred antioxidants for an LMBW include, but are not limited to, EDTA, citric acid, glycine, butylated hydroxyanisole, butylated hydroxytoluene, cysteine, methionine, thioglycerol, bisulfite, metabisulfate, ascorbate, propyl gallate, tocopherol, reducing sugar, or salt or mixture thereof.

As used herein, "aqueous" means that the composition is made with water as the main solvent with its concentration greater than the concentration of any other liquid solvent.

As used herein, "alcohol" is an organic solvent in which one hydroxyl functional group (-OH) is bound to a carbon atom and this carbon is saturated, having single bonds to three other atoms. In an LMBW, an Alcohol is an excipient added primarily to increase solubility of a drug. The alcohols useful for an LMBW are ethanol, benzyl alcohol, propylene glycol, polyethylene glycol of a low molecular weight (100-5,000) or a combination thereof.

As used herein, the term "Alcohol to Dry Weight Ratio" or "Alcohol/LBM" refers to the ratio of Alcohol weight to the Dry Weight in an LMBW. For example, for an LMBW composition comprising 61 g lecithin, 21 g MCT, 18 g bile salt and 49 g alcohol, the Dry Weight is 100 g (= 61g + 21g + 18g) and the Alcohol to Dry Weight Ratio or Alcohol/LBM is 49/100 or 0.49/1.

As used herein, a "base" is an alkalizing agent used to adjust the pH of an LMBW. Both organic and inorganic bases can be used. The preferred bases include, but are not limited to, sodium hydroxide, potassium hydroxide, calcium hydroxide, ammonium hydroxide, lysine, arginine, or a combination thereof.

As used herein, a "bile salt" or "B" used in an LMBW composition refers to an alkali salt, such as sodium salt, of cholic acid, glycocholic acid, taurocholic acid, deoxycholic acid, glyco- or taurodeoxycholic acid, chenodeoxycholic acid and glyco- or taurochenoxydeoxycholic acid. Sodium glycocholate and sodium deoxycholate are preferred bile salts. Other cations including, but not limited to, potassium, lithium, calcium, arginine, lysine or ammonium salt may also be used in a bile salt. For use in an LMBW, either a bile acid or a bile salt can be used as a starting material. If a bile acid is used as the starting material, it is usually converted (completely or partially) to its bile salt (i.e., the conjugate base) by adjusting pH upward before or during the preparation of an LMBW. If a bile salt is used as the starting material, it may be converted (completely or partially) to its bile acid (i.e., the conjugate acid) by adjusting pH downward during the preparation of an LMBW. In one aspect, the term "bile salt" or "B" used in this application also refers to a mixture of a bile salt and its conjugate acid. For example, the pKa of deoxycholic acid is 6.58 (The Merck Index, 12^{th} Ed., Entry #12946). According to the Henderson-Hasselbalch equation, a solution of deoxycholic acid in water at pH 4.58 would contain a mixture of 99% protonated deoxycholic acid (bile acid) and 1% deprotonated deoxycholate (bile salt). Such a bile acid/bile salt mixture is well suited for use in an LMBW. For clarity, concentration of a bile salt used in an LMBW is provided herein as the concentration of the conjugate bile acid.

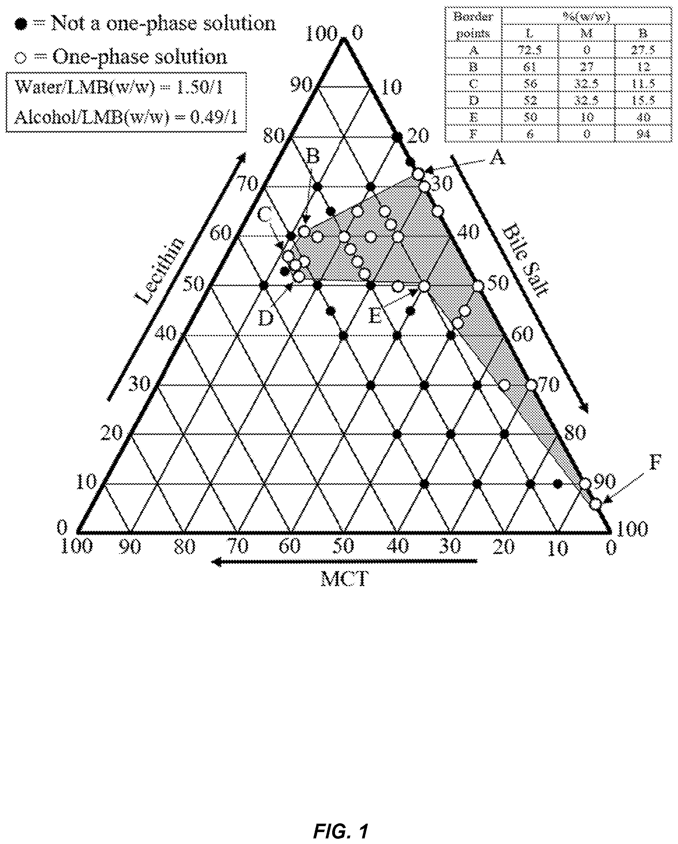

As used herein, a "Border point" refers to an LMBW composition which is very close to a non-LMBW composition in a ternary phase diagram (Example, FIGS. 1-5). Each Border point is experimentally determined and is used together with other Border points to map out the "LMBW zone" within which all compositions are clear and one-phase, i.e. an LMBW, in the ternary phase diagram.

A "bulking agent" of this invention refers to an excipient that is added to an LMBW composition to protect its components during and after freezing or drying (usually by lyophilization) and to provide the solid bulk after the drying. The bulking agents that are useful for an LMBW include, but are not limited to, monosaccharides, disaccharides, polysaccharides, propylene glycols, polyethylene glycols, glycerols, poly-ols, dextrins, cyclodextrins, starches, celluloses and cellulose derivatives, proteins, peptides, amino acids, sodium chloride, polyvinypyrrolidone, or mixtures thereof. The preferred bulking agent for an LMBW is a carbohydrate such as mannitol, sorbitol, xylitol, lactose, fructose, xylose, sucrose, trehalose, mannose, maltose, dextrose, dextran, or a mixture thereof. The concentration of the bulking agent useful in an LMBW may be from about 1% to about 30% w/w, preferably, from about 2% w/w to about 10% w/w or more preferably, from about 3% w/w to 8% w/w, based on weight of the LMBW composition.

As used herein, the terms "clear" and "transparent" are used herein interchangeably and mean that the composition has a light transmittance value measured at 800 nm light wavelength (T800) using a 10-mm quartz cuvette by a UV-vis spectrophotometer exceeding 70%, preferably 80% and more preferably 90%.

With such high light transmittance, it is possible to see through the clear composition in a clear glass vial, which allows for examination of presence of foreign particles (e.g. broken glass or metal particles) or other abnormal defects (e.g. microbial growth) that are unsafe for injection. In contrast, a multiple-phase injectable composition such as DIPRIVAN^{®} (emulsion with T800 value of less than 10%) and EXPAREL^{®} (liposome with T800 value of less than 70%) are white, opaque or hazy liquid and do not permit good visual examination for the harmful foreign particles.

As used herein and unless otherwise stated, the term "concentration" refers to the weight percent concentration based on the Dry Weight in a composition of interest. For example, for a composition containing 20 g of L, 6 g of M and 11 g of B, the Dry Weight is 37g (= 20 + 6 + 11) and the concentrations for L, M and B, are 54.1% (= 20/37), 16.2% (= 6/37) and 29.7% (= 11/37), respectively.

As used herein, diazepam, also known as Valium^{®}, is a medicine of the benzodiazepine family that typically produces a calming effect. It is commonly used to treat a range of conditions including anxiety, alcohol withdrawal syndrome, benzodiazepine withdrawal syndrome, muscle spasms, seizures, trouble sleeping, and restless legs syndrome. It is used in emergency situations to stop cluster seizures (episodes of increased seizure activity). A diazepam rectal gel (Diastat^{®}) is approved by the FDA for this emergency or rescue use, but the rectal gel is inconvenient to use. A ready-to-use and preferable self-administered injection is a better dosage form. Diazepam is very insoluble in water, the currently marketed diazepam injection formulation (Diazepam Injection, USP) can dissolve only about 5 mg/mL diazepam. For the seizures rescue dose of 20 mg, a 4-mL injection volume would be required if the current marketed formulation is used, which is too much volume for intramuscular or subcutaneous injection. Therefore, there is a need to develop a new injectable diazepam formulation, which can significantly increase diazepam solubility and allow for delivery of 20 mg diazepam in about 1-2 mL volume. An LMBW composition capable of solubilizing diazepam to 17 mg/mL and delivering 20 mg in 1.2 mL volume was thus developed and tested in human with excellent safety outcomes (Example 25).

As used herein, the "drug" means any chemical that is biologically active.

As used herein, the phrase "Dry Weight" refers to the total weight of L, M and B in a composition of interest in this invention. Dry Weight does not include any other component that may also be present in the composition. For example, for a composition containing 61 g L, 21 g M, 18 g B, 50 g alcohol, 1 g preservative, 1 g antioxidant and 150 g water, the Dry Weight is 100 g (= 61 + 21 + 18). Dry Weight is used as the weight base to calculate the weight percent concentration of L, M or B in a composition of interest in this invention.

As used herein, an "excipient" refers to a biologically inactive substance that serves various functions in a drug formulation such as a vehicle, medium, stabilizer, preservative etc. An LMBW is a uniquely defined combination of 4 excipients (L, M, B and W). Other excipients useful in an LMBW may include but not limited to alcohol, pH buffer, osmotic adjusting agent, preservative, antioxidant, bulking agent.

As used herein, an "emulsion" is a mixture of two liquids that are normally immiscible (unmixable or having two phases). In an emulsion, one liquid (the dispersed phase) is dispersed in the other (the continuous phase). Two liquids can form different types of emulsions. As an example, oil and water can form, first, an oil-in-water emulsion, wherein the oil is the dispersed phase, and water is the dispersion phase. Second, they can form a water-in-oil emulsion, wherein water is the dispersed phase and oil is the dispersion phase. For injectable drug formulations, the oil-in-water type is more common. In an oil-in-water emulsion, the oil phase exists as small oil droplets in 50-5000 nm diameter suspended in water (the aqueous phase). For example, the droplet size of the injectable emulsion drugs DIPROVAN^{®} (Propofol emulsion for injection) and INTRALIPID^{®} are about 150-500 nm in diameter, both contain lecithin and oil, and both are white and opaque with T800 less than 10%. The LMBW compositions of the present invention are not emulsions.

As used herein, "formulation" refers to a mixture of excipients or other chemicals prepared according to a specific recipe and preparation procedure. A formulation constitutes the final or intermediate form of a drug, cosmetic or supplement product, such as tablet, capsule, lotion etc. A formulation may optionally contain a drug. The terms of "composition" and "formulation" are used interchangeably in this application.

As used herein, "FDA" refers to the U.S. Food and Drug Administration.

As used herein, "filterable" means the ability of a liquid to pass through a filter membrane of a certain pore size such as 0.2 microns. The LMBW compositions of the present invention are filterable.

The term of "high-energy homogenization" used herein refers to any homogenization method using equipment, which employs high-pressure or ultrasonification mechanism, to render particle reduction and mixing. A high-pressure homogenizer works by using a high pressure (e.g. >10,000 psi) to force a liquid sample through a small channel to generate high shear effect, which breaks up the particles or oil into small particle/droplets. Examples of high-pressure equipment include, but not limited to, microfluidizers, APV homogenizers, Gaulin homogenizers and Rannie homogenizers. Ultrasonification homogenizer, referred herein, may include probe type or bath type. The formation of LMBW compositions of the present invention does not need high-energy homogenization.

The "low energy mixer" used herein refers to a rotational stirrer operating at a speed of less than 5000 RPM or a one- or multi-dimensional shaker operating at frequency of less than 5000 cycles per minute. The rotational stirrer may include, but not limited to, a simple propeller or impeller type mixer or a rotor-stator type of high shear mixer. A high shear mixer uses a rotating impeller or high-speed rotor, or a series of such impellers or inline rotors, usually powered by an electric motor, to "work" the fluid, creating flow and shear. A stationary component may be used in combination with the rotor and is referred to as the stator. The stator creates a close clearance gap between the rotor and itself and forms a high shear zone for the material as it exits the rotor. The formation of LMBW compositions of the present invention may be facilitated by a low energy mixer.

As used herein, the word "injectable", or phrase "suitable for injection" refers to a formulation that can be injected through a hypodermic needle safely into human or animal patients when (1) each of the components in the formulation has been used in a marketed injection drug approved by the FDA at the time of this application, (2) the formulation can be expelled manually using a syringe through a needle smaller than 21 gauge, preferably 25 gauge and more preferably 27 gauge, (3) the formulation is free of any particles of size > 10 micron in diameter, and (4) the viscosity of the formulation is less than 10,000 centipoises, preferably less than 1,000 centipoises and more preferably less than 100 centipoises.

As used herein, the word "injection" means insertion of liquid into the body via a hypodermic needle into one or more different locations that include, but are not limited to, intravenous, intra-arterial, subcutaneous, intramuscular, intradermal, intracavernous, intracavitary, intravesical, interstitial, intra-articular, intradermal, intrabursal, intralesional, intratumor, intraperitoneal, intrapleural, intrasynovial, intrathecal, and intratracheal, or via infiltration, nerve block, IV infusion, or other route of injection.

As used herein, an "insoluble drug" refers to a drug or pharmacologically active agent lacking solubility in aqueous solutions (such as water, physiological saline, injectable dextrose solutions, etc.). The USP/NF generally expresses the solubility in terms of the volume of solvent required to dissolve 1 gram of the pharmacologically active agent at a specified temperature (e.g., 1 g aspirin in 300 ml water, 5 ml ethanol at 25°C). Other references may use more subjective terms to describe solubility, such as those given in the following table from Remington's Pharmaceutical Sciences, Mack Publishing Co., Easton, Pa., latest edition.

| Descriptive terms | Parts of solvent needed for 1 part solute |
|---|---|
| Very soluble | <1 |
| Freely soluble | 1-10 |
| Soluble | 10-30 |
| Sparingly soluble | 30-100 |
| Slightly soluble | 100-1000 |
| Very slightly soluble | 1000-10,000 |
| Practically insoluble or insoluble | >10,000 |

Thus, as used herein, an "insoluble drug" of this invention includes any drug whose solubility would place it in the bottom four solubility categories in the above table, i.e., "sparingly soluble," "slightly soluble," "very slightly soluble," and "practically insoluble or insoluble," when water is used as the solvent. As used herein, the word "insoluble" may be used interchangeably with hydrophobic, lipophilic, oleophilic, and other similar terms. Examples of insoluble drugs that can be dissolved by an LMBW include, but are not limited to amiodarone, amphotericin, aprepitant, camptothecin, clarithromycin, cyclosporine, diazepam, docetaxel, ibuprofen, celecoxib, itraconazole, irinotecan, iodine, levodopa, paclitaxel, phytonadione, posaconazole, prednisolone acetate, progesterone, propofol, resveratrol, and voriconazole.

As used herein, the phrase "IV infusion fluids" refer to the sterile aqueous solutions commonly used to dilute a drug for intravenous infusion. Examples of IV infusion fluids useful to dilute an LMBW include, but are not limited to, 5% Dextrose Injection, USP, 5% Dextrose Injection, USP and 0.9% Sodium Chloride Injection, USP, Lactated Ringer's Injection, USP, 5% Dextrose and Lactated Ringer's Injection, USP, Normosol-M and 5% Dextrose, 0.9% Sodium Chloride Injection, USP, and ISOLYTE E.

As used herein, the phrase "laser light scattering" or "dynamic light scattering (DLS)" is a technique in physics that can be used to determine the size distribution profile of small particles in liquid, including proteins, polymers, micelles, mixed micelles, liposomes, emulsions, vesicles, carbohydrates, nanoparticles, biological cells and gels. A DLS is one of the most sensitive tools to detect small particles. For example, with a sizing range between 0.6 nm to 10 microns, the DLS spectrometry used to examine the LMBW composition (see Example 13 & Example 16 Malvern Zetasizer, Model Nano-ZS) is capable of detecting any nanometer- and micrometer-sized particles, droplets or supramolecular assembly such as liposomes, emulsions, micelles, mixed micelles or nanoparticles.

As used herein, "long chain triglycerides" or "LCT" are tri-esters of glycerol with saturated and unsaturated fatty acids having long chains of 18 to 24 carbons in length. LCTs are found in most foods and are abundant in nature. LCTs are the main components of vegetable oils such as sesame oil, soybean oil, corn oil, safflower oil, castor oil, olive oil, etc. Interestingly, it was discovered by the inventors that LCT or a vegetable oil such as sesame oil cannot form an LMBW. Thus, in the present invention, the MCT cannot be replaced by an LCT.

As used herein, "lecithin" or "L" is a mixture of phospholipids derived from a natural source. Injectable lecithin includes lecithin which has been derived from eggs, soybeans, sunflower seeds or other natural sources, further purified and is substantially free from proteins or other irritating, allergenic, inflammatory agents or agents that cause other deleterious biological reactions. For this invention, the preferred lecithin includes those that contain more than 75% (w/w over the total weight of lecithin) phosphatidylcholine (PC). Examples of the preferred lecithin include, but are not limited to, commercial lecithin products going by the trade names of LIPOID S 75, LIPOID S 100, LIPOID E 80, and Phospholipon 90 G. Lecithin useful for an LMBW composition may also include synthetic phospholipids, preferably a PC or a mixture of synthetic phospholipids having more than 75% (w/w over the total weight of the mixture) PC.

The term "light transmittance (%)" used herein is a measurement of transparency of a liquid and is defined as the fraction of incident light at a specified wavelength (i.e., 800 nm or *lambda)* that passes through a sample contained in a transparent cuvette (e.g. a quartz cuvette with 10 mm path length). It is calculated using the following equation: ${T}_{\left(lambda\right)} = I \div {I}_{0} \times 100$ **where** *I*₀ is the intensity of the incident light and *I* is the intensity of the light coming out of the sample and *T_{(lambda)}* is transmittance at the wavelength lambda. The *T_{(lambda)}* value can be readily measured by a UV-visible spectrophotometer at a fixed wavelength. For the clear compositions of this invention, T800 is a T _{(lambda)} value measured at 800 nm in a quartz cuvette with 10 mm path length using a UV-visible spectrophotometer.

As used herein, a "liposome" is a spherical nanometer- and micrometer-sized solid particle having a bilayer membrane formed by lecithin or phospholipid molecules. The selforganizing bilayer membranes are the characteristic structural feature of liposomes. Liposomes are most often composed of phospholipids, especially phosphatidylcholine, but may also include other lipids, such as egg phosphatidylethanolamine, so long as they are compatible with lipid bilayer structure. The major types of liposomes are the multilamellar vesicle (MLV, with several lamellar phase lipid bilayers), the small unilamellar liposome vesicle (SUV, with one lipid bilayer), the large unilamellar vesicle (LUV), and the cochleate vesicle. Liposomes should not be confused with micelles or mixed micelles which are composed of monolayers. A liposome particle size can range from 20 nm to 40 µm in diameter. For example, the liposome particle size in the liposome drugs AmBisome^{®}, Doxil^{®}, Caelyx^{®} and EXPAREL^{®} are reportedly 77.8 nm, 80-85 nm, 80-85 nm and 24-31 µm, respectively. A liposome formulation or liposome composition is often referred to an aqueous suspension of the liposome particles. Clearly, the liposome formulation or liposome composition is a 2-phase system and is usually hazy or not clear in appearance due to the presence of the particles. The LMBW compositions of the present invention are not liposomes.

As used herein, an "LMBW" refers to a composition comprising Lecithin, Medium chain triglyceride (MCT), and Bile salt constructed in quantities within an LMBW Zone.

As used herein, the term "LMBW Properties" refers to the following desirable basic physical and biological properties of an LMBW that include being (1) a clear and one-phase solution, (2) capable of dissolving insoluble drugs of widely different structures, i.e. a Universal Solubilizer, (3) spontaneously self-forming in water (i.e., does not require high-energy homogenization for its preparation), (4) can be further diluted in water or an IV infusion fluid without any precipitation or phase separation i.e., remains as one-phase solution after being diluted with water, and (5) injectable.

As used herein, an "LMBW zone" refers to a zone in a ternary phase diagram having L, M, B in various combinations and water at a fixed water to Dry Weight Ratio. The LMBW zones were experimentally determined and systematical mapped out by the inventors of this application. Examples of LMBW zones are graphically shown in FIGS. 1-5. An LMBW zone is used to define the upper and lower concentration limits for each of the L, M and B components. Only within the LMBW zone, a mixture of L, M and B can form a clear and one-phase solution in water or an LMBW.

As used herein, a "Medium Chain Triglycerides", "MCT" or "M", which are also known as "medium chain triglyceride oil" are tri-esters of glycerol with fatty acids having medium length chains of 6 to 12 carbons in length. An MCT can be either derived from a natural source or made synthetically. Natural MCT is commonly derived from coconut oil and contains a mixture of glyceryl tri-esters of fatty acids with different chain lengths (6-12 carbons C₆, C₇, C₈, C₉, C₁₀, C₁₁ and/or C₁₂), or mono-, di- and tri-glycerides. Examples of the natural MCT include commercial products with the trade names CRODAMOL GTCC-PN, Miglyol 812, CAPMUL MCM and Neobees M-5. A synthetic MCT may contain essentially one molecule. Examples of synthetic MCT may include, but are not limited to, glyceryl tri-esters of propionic acid, butyric acid, valeric acid, caproic acid, enanthic acid, caprylic acid, pelargonic acid, capric acid or the mixture thereof. Examples of the preferred MCTs for an LMBW include Miglyol 812 and synthetic triglycerides having 6, 8, 10 or 12 carbon fatty acids.

As used herein, a "micelle" is an aggregate or supramolecular assembly with single layer membranes of surfactant molecules dispersed in a liquid such as water. A typical micelle in aqueous solution forms an aggregate with the hydrophilic "head" regions in contact with water, sequestering the hydrophobic single-tail regions in the micelle center. Micelles are generally formed by water soluble surfactant with an HLB or Hydrophilic-Lipophilic Balance value greater than 7. Lecithin alone generally cannot form micelles in water because it is insoluble in water (it forms liposome, solid lipid particles or emulsion when combined with oil). However, when combined with a water-soluble surfactant, lecithin can form mixed micelles in water.

As used herein, a "mixed micelle" is an aggregate or supramolecular assembly with single layer membranes of lecithin by incorporating itself into a water-soluble surfactant micelle in water. Mixed micelles made with combination of a bile salt (which is a water-soluble surfactant) and lecithin have been used to solubilize drugs. For example, VALIUM^{®} MM (diazepam injection) and KONAKION^{®} MM (vitamin K injection) are lecithin-bile salt based mixed micelle (MM) formulation. The particle size of the lecithin-bile salt mixed micelles has been reported to be about 8 nm or greater in diameter as measured by various analytical techniques including laser light scattering (J. Wylie Nichols and Justyna Ozarowski, "Sizing of lecithin-bile salt mixed micelles by size-exclusion high-performance liquid chromatography" Biochemistry 1990, 29, 4600-4606), or an average size from ~5 nm to ~ 14 nm depending upon the bile salt concentration as confirmed by another group of researchers (Biochimica et Biophysica Acta 1808 (2011) 140-153). Unlike an LMBW which is a one-phase solution, presence of these 5 nm or greater particles in a mixed micelle composition clearly indicate that it is not a one-phase solution.

As used herein, an "osmotic adjusting agent" refers to an ingredient added to a composition of this invention to increase its osmotic pressure. An LMBW may have an osmotic pressure of approximately 100 to 1000 mOsm with the preferred range of 200 to 400 mOsm such as 225, 250, 275, 300, 325, 350, or 400 mOm and most preferred value of about 300 mOsm. Useful osmotic adjusting agents for an LMBW include, but are not limited to, potassium or sodium chloride, trehalose, sucrose, sorbitol, glycerol, mannitol, polyethylene glycols, propylene glycol, albumin, amino acids and mixtures thereof.

As used herein, the term "one-phase" describes a liquid which is substantially free of solid particles, oil droplets, or other detectable aggregates or super molecular structures in the nanometer (> 5 nm and ≤ 1000 nm), micron or micrometer (>1 micron and ≤ 1 millimeter) or greater (> 1 millimeter) size range. Any object in water of size less 5 nm falls in the size range of single or un-aggregated molecules, therefore is regarded as a true solution or one-phase solution.

As used herein, "pH" is a measure of the acidity or basicity of an aqueous solution. The pH determination of a composition of the present invention is typically performed with a pH meter consisting of a glass electrode connected to an electronic meter that measures and displays the pH readout. The pH meter is calibrated using standard pH buffers. Solutions with a pH less than 7 are said to be acidic and solutions with a pH greater than 7 are basic or alkaline. Pure water (i.e., pure H₂O) has a pH close to 7.

As used herein, a "pH buffer" means the common pH buffers used in the food, cosmetic or pharmaceutical formulations used to stabilize the pH of a solution. The preferred buffers for an LMBW include, but are not limited to, phosphate, acetate, citrate, TRIS, bicarbonate, succinate, histidine, or other amino acids.

As used herein, a "phase diagram" or "ternary phase diagram" is an equilateral triangle plot graphically depicting concentrations of the three components of interest in a combination. For the LMBW compositions of this invention, the three components are L, M and B. FIGS. 1-4 are examples of the ternary phase diagrams for L, M and B. Each axis represents the concentration (in weight percentage based on the Dry Weight) of one component. Each point in a ternary phase diagram represents a unique composition containing L, M and B. For example, the point "E" in FIG. 1 represents a combination containing 50% L, 10% M and 40% B based on the Dry Weight. The purpose of ternary phase diagram in this invention is to provide a visual & quantitative representation of the LMBW zone which was experimentally determined. With an LMBW zone displayed in a ternary phase diagram, one can readily check if a given mixture of L, M and B is within an LMBW zone, and if so, expect such mixture to form a clear and one-phase solution in water and to have the LMBW properties.

As used herein, a "preservative" is a pharmaceutical additive that can be added to a composition to inhibit bacterial and fungal growth. The antimicrobial preservatives useful in an LMBW include, but are not limited to, cresols, phenol, benzyl alcohol, ethanol, chlorobutanol, parabens, imidura, benzylkonium chloride, EDTA or its salt, or a combination thereof.

As used herein, the term "Solid LMBW" refers to a LMBW composition that exists in a solid form. Typically, a Solid LMBW contains a low water with a Water to Dry Weight ratio of less than 0.1/1 and is substantially from of alcohol. A Solid LMBW is an amorphous transparent glass-like solid having a different physical structure than its starting components (L, M, and B) if they are directly mixed together ("physical mixture") (Example 28). A Solid LMBW can be prepared by making a LMBW solution with water and/or alcohol first and then removing the water and alcohol by vacuum drying or other drying methods to reach the final water content, i.e. Water to Dry Weight ratio of less than 0.1/1. A Solid LMBW can be used to solubilize a drug and allow for incorporation into a solid dosage form such as tablet or capsule. Upon mixing with water, A Solid LMBW can be readily converted into a regular LMBW solution having the same LMBW properties.

As used herein, the term "solubility" means that a solute (such as a drug) has reached its maximum concentration in a solvent.

As used herein, the term "solubilizer" or "solubility enhancer" refers to an excipient or a combination of excipients that is used to increase the solubility of a compound of interest in water. The compound of interest may include, but not limited to a drug, a cosmetic agent, a nutritional supplement, an endogenous body component such as cholesterol, and mixture thereof.

As used herein, the term "substantially free" means containing less than 1% of the total weight of the composition of interest.

As used herein, "solution" refers to a one-phase liquid.

As used herein, the term "Universal Solubilizer" refers to a solubilizer that is capable of increasing the solubility for a compound of interest regardless of its structure, MW or physicochemical properties. More specifically, the term "Universal Solubilizer" refers to a solubilizer that is capable of increasing the solubility to ≥ 1 mg/mL of an inorganic element, small or non-polymeric organic molecule, peptide, or protein in water. The ≥ 1 mg/mL concentration is regarded as a useful concentration for pharmaceutical applications for most drugs.

As used herein, "USP" means the current edition of the United States Pharmacopeia.

As used herein and unless otherwise stated, the term "%" means the weight by weight percentage, %wt or %w/w.

As used herein, the term "Water to Dry Weight ratio" or "Water/LBM" refers to the weight ratio (w/w) of water to Dry Weight in an LMBW or a L, M and B composition of interest. For example, for a composition containing 61 g L, 21 g M, 18 g B and 150 g water, the Dry Weight is 100 g (= 61g + 21g + 18g) and the Water to Dry Weight ratio or Water/LBM ratio is 150/100 or 1.5/1.

### II. Description of Embodiments

It is widely known that lecithin is a useful solubilizer for insoluble drugs. In pharmaceutical formulation practice, lecithin has been used to form emulsion, liposome, mixed micelle and nanoparticle formulations. The most commonly used lecithin in drug formulations are mixtures of phospholipids derived from a natural source, typically soy beans or egg yolk. The soy or egg lecithin itself is insoluble in water. In water, lecithin generally forms a two-phase system such as an emulsion (with oil), liposome, mixed micelle (with a soluble surfactant), or nanoparticle suspension.

Lecithin can form oil-in-water or water-in-oil emulsions. Injectable emulsion compositions are generally oil-in-water type consisting of 3 members consisting of lecithin, oil and water (which is unlike an LMBW having four components L, M, B and W). Emulsions are two-phase systems consisting of an aqueous phase and oil droplets with size generally about 50-5000 nm. An injectable emulsion is white and opaque with T800 generally less than 50%. In contrast, an LMBW is clear with T800 generally exceeding 90% (Example 16). In addition, an LMBW is a one-phase solution without any oil droplet of size greater than 5 nm (Example 16) and can dissolve significantly more of an insoluble drug than an emulsion composition having the same lecithin concentration (Example 2).

Lecithin can also form liposomes in water. Liposomes are nanometer- and micrometer-sized solid particles or vesicles having a bilayer membrane formed by the lecithin phospholipid molecules. The bilayer membranes are the characteristic structural feature of liposomes. A liposome particle can range in the size from 20 nm to 40 µm in diameter. A water composition containing liposomes is clearly a 2-phase system or a suspension, whereas an LMBW is a one-phase solution without any solid particles of size greater than 5 nm (Example 16).

Lecithin can't form a clear solution or dispersion in water because it is insoluble in water. It, however, can form mixed micelles in water when combined with a water-soluble surfactant such as a bile salt. Micelles is a distinct and separate phase in water. The particle size of the lecithin-bile salt mixed micelles has been reported to be about 8 nm or greater in diameter as measured by various analytical techniques including laser light scattering (J. Wylie Nichols and Justyna Ozarowski, "Sizing of lecithin-bile salt mixed micelles by size-exclusion high-performance liquid chromatography" Biochemistry 1990, 29, 4600-4606), or an average size from 5 nm to 14 nm depending upon the bile salt concentration as confirmed by another group of researchers (Biochimica et Biophysica Acta 1808 (2011) 140-153).

In comparison, an LMBW does not contain any particles of greater than 5 nm (Ex 16). Mixed micelle compositions are ternary (3 members) mixtures consisting of lecithin, bile acid and water, whereas an LMBW is a quaternary (4 members) mixture consisting of four components: lecithin, bile acid, MCT and water. In addition, an LMBW can dissolve significantly more quantities of an insoluble drug than a mixed micelle composition having the same bile salt and lecithin concentrations (Example 2).

Lecithin has also been used to form suspensions in water where the lecithin phospholipids are homogenized in water to form small phospholipid particles (US Pat. No. 5785976A). In some cases, these suspended lecithin particles are also referred to as solid lipid particles of size about 20-500 nm A suspension is a 2-phase liquid.

All these lecithin-based compositions (emulsions, liposomes, mixed micelles, solid lipid particles) share the common structural feature of having two or more phases, and these two-phase systems are not self-forming and require high energy input for their formation. Therefore, they are physically unstable and would degrade (particle aggregation, phase separation) over time, which makes them less desirable for drug formulations.

Lecithin can be dissolved in oils, such as a vegetable oil, or alcohol to form a clear and one-phase solution. However, such an oil or alcohol solution of lecithin will precipitate or turn into a cream/paste-like mass immediately upon dilution with water, rendering it unsafe for intravenous injection. In contrast, an LMBW is an aqueous solution and remains as a clear and one-phase solution upon dilution with water.

The above lecithin-based but non-LMBW formulations have limited solubility enhancement potential (Example 2). For many insoluble drugs, such formulations are not pharmaceutically useful because they cannot dissolve enough drug to meet the therapeutic dose requirement.

In summary, none of these commonly employed lecithin-based drug formulations share the same structural features and physical properties as an LMBW, and an LMBW is generally superior to other lecithin-based formulations as a solubilizer.

As will be shown in the Examples below, LMBW formulations have the physical and biological properties that are very desirable for therapeutic applications, especially for formulating insoluble drug compounds. These LMBW properties are:
1. being a clear and one-phase solution,
2. can be further diluted in water or in an IV infusion fluid without any precipitation or phase separation (i.e., remains as one-phase solution after being diluted with water),
3. capable of dissolving insoluble drugs of widely different structures, i.e. a Universal Solubilizer,
4. forms spontaneously or is self-forming in water (i.e., does not require highshear/high-pressure homogenization for its preparation), and
5. is safe for injection into human or animals, or injectable.

To find all possible mixtures or compositions of L, M, B that can form an LMBW, the inventors of this application have systematically prepared numerous compositions containing L, M, and B in water and examined each composition prepared by using visual, microscopic and/or dynamic light scattering spectrometry method to determine whether the composition was a clear and one-phase solution. Once confirmed to be clear and one-phase, the composition was further diluted in water and examined again for clarity and phase separation. If the composition remained clear and one-phase, it was marked as an LMBW with an open circle on a ternary phase diagram (Example 1).

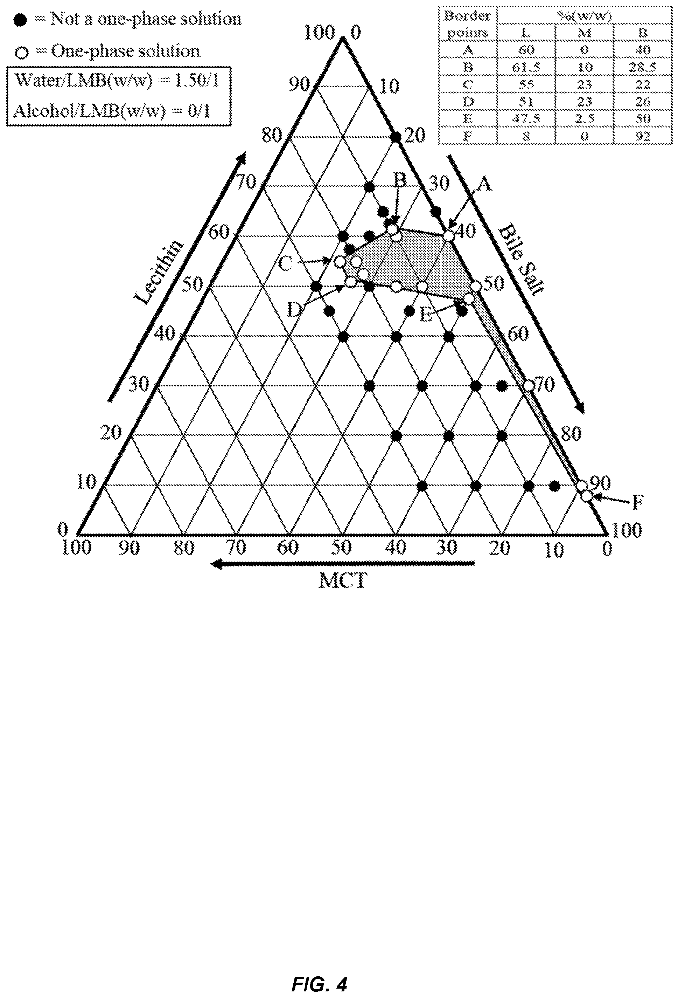

Each prepared and tested composition is quantitatively represented by the concentrations of L, M and B (in weight percent concentration based on Dry Weight) and is depicted graphically by an open or black circle in a ternary phase diagram (see FIGS. 1-4). All compositions in a ternary phase diagram were prepared at a fixed Water to Dry Weight ratio and optionally a fixed Alcohol to Dry Weight ratio. For FIG. 4, no alcohol was added in any composition (i.e., Alcohol to Dry Weight ratio = 0/1).

The open circles represent compositions that produced clear and one-phase solutions at the fixed water and alcohol levels and remained clear and one-phase after further dilution in water, whereas the black circles represent compositions that were not clear or failed to exist as a single phase.

Special efforts were made to prepare, test and ascertain the open circles that are graphically very close to the black circles. Once confirmed, these open circles were labeled as "Border points". By connecting the Border points to form an enclosed section, an LMBW zone is graphically defined or "mapped out" in the ternary phase diagram (see shaded areas in FIGS. 1-5).

In each LMBW zone shown in FIGS. 1-4, the water content or the Water to Dry Weight ratio was fixed at 1.5/1. For each LMBW zone, the water content may increase freely upward, because any composition in the LMBW zone can be diluted with water without losing the LMBW property. Similarly, an LMBW composition may be diluted with an IV infusion fluid such as Normal Saline or 5% dextrose solution to a high extent (e.g. 10X or 100X) without precipitation or phase separation (Example 11). A diluted LMBW (by water or an IV infusion fluid) would retain the LMBW properties.

It was also discovered by the inventors of this invention that an LMBW requires a minimum amount of water to form. The minimum amount of water was determined to be about 0.1/1 (in Water/Dry Weight ratio). Once the water content was reduced to below 0.1/1, an LMBW would turn into a solid (Example 14 & 18).

Alcohol can be added to an LMBW as an optional excipient. The primary purpose of adding alcohol is to further increase the solubility of an insoluble drug in the LMBW. Moreover, alcohol can reduce the viscosity of an LMBW, making it easier to inject or transfer. Some alcohols such as benzyl alcohol can also function as a preservative which will allow an LMBW formulation to be suitable for multiple injections or dosing.

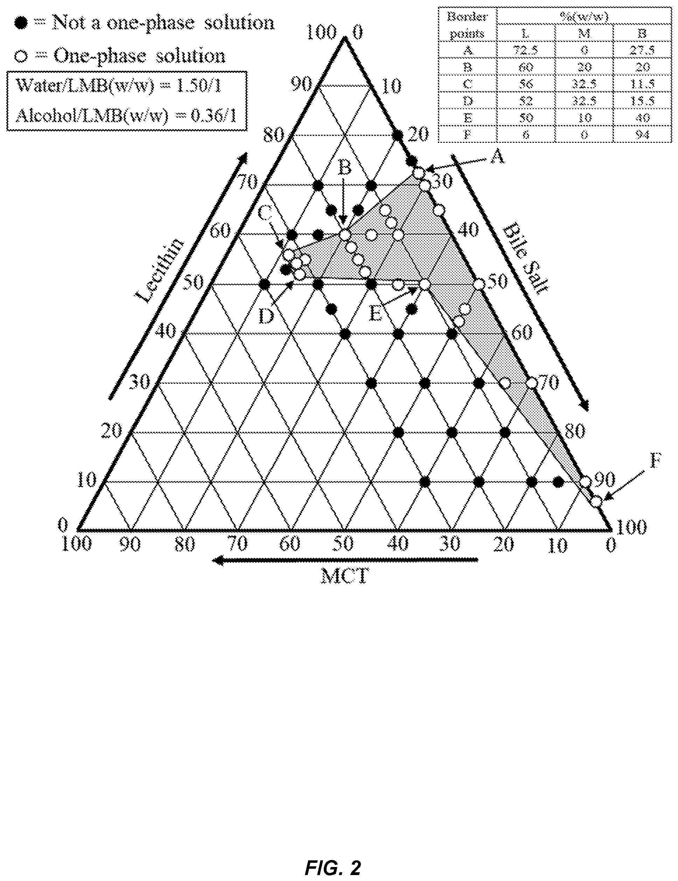

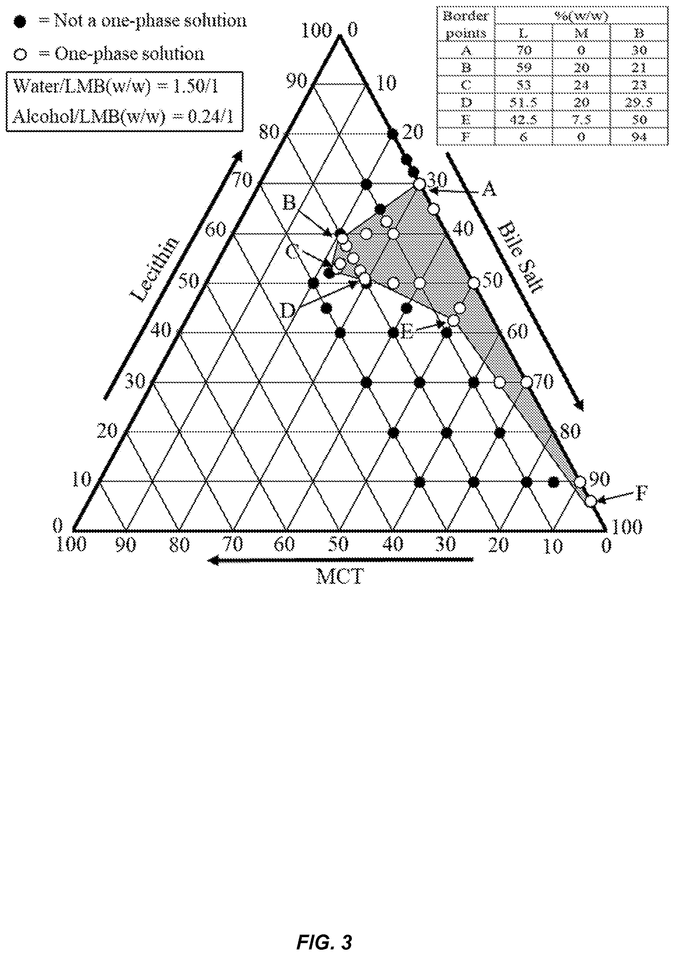

As the alcohol concentration increases, the area of the LMBW zone will also increase. For example, the area of the LMBW zone in FIG. 1 (with 0.49/1 in Alcohol to Dry Weight ratio) is greater than that in FIG. 3 (with 0.24/1 in Alcohol to Dry Weight ratio). However, it appeared that the LMBW area would reach a maximum at 0.49/1 (in Alcohol to Dry Weight ratio), as further increase in alcohol concentration from 0.36/1 (FIG. 2) to 0.49/1 (FIG. 1) did not result in a significant increase in the LMBW zone. Therefore, it is believed that the LMBW zone shown in FIG. 1 (or described in Claim 1 & 2) represents the greatest LMBW zone possible.

In one aspect, the greatest LMBW zone contains from about 6% to about 72.5% L, such as 6%, 10%, 15%, 20%, 25%, 20%, 35%, 40%, 45%, 50%, 55%, 60%, 65%, 70% or about 72.5% L; between 0% and about 32.5% M, such as 0.1%, 2%, 5%, 10%, 15%, 20%, 25%, 30% or about 32.5% M, and from about 11.5% to about 94% B, such as 11.5%, 15%, 20%, 25%, 30%, 35%, 40%, 45%, 50%, 55%, 60%, 65%, 70%, 75%, 80%, 85%, 90% or about 94 % B in water.

In another aspect, the greatest LMBW zone is within (but not including Border points where the value is 0) the section enclosed by the following Border points:

| Border points (as in FIG. 1) | Concentration (% w/w based on the Dry Weight) | | |
|---|---|---|---|
| | Lecithin | MCT | Bile salt |
| A | 72.5 | 0 | 27.5 |
| B | 61 | 27 | 12 |
| C | 56 | 32.5 | 11.5 |
| D | 52 | 32.5 | 15.5 |
| E | 50 | 10 | 40 |
| F | 6 | 0 | 94 |

In FIG. 1, each side or axis denotes the concentration of the labeled component. The arrow under each axis label points to the angle which represents 100% w/w of the labeled component. For example, the top angle represents a composition that is 100% w/w in Lecithin and 0% in Bile salt. Similarly, the lower and right angle represents 100% w/w in Bile salt and 0% w/w in MCT and the lower left angle 100% w/w MCT and 0% w/w Lecithin. Any point on an axis is binary mixture such as the Border points A & F. Each point inside the triangle represents a ternary mixture. For example, Border point B contains 61% w/w L, 27% M and 12% B. The purpose of the ternary phase diagram is to provide a visual and quantitative presentation of the LMBW zone. An LMBW zone is the grey area enclosed by the Border points (open circles). The lines connecting the Border points form the borders that separate the LMBW zone from the non-LMBW zones, which are mapped out by the solid circles. Each open circle or solid circle composition was experimentally prepared and tested to determine whether it is a clear and one-phase solution. It is worthwhile to note that any composition that falls on the A-F line cannot be regarded as an LMBW since MCT is absent.

With an LMBW zone displayed in a ternary phase diagram, one can readily check if a given mixture of L, M and B can form an LMBW, and if so, expect such mixture to have the LMBW properties. For each ternary phase diagram (FIGS. 1-4), the water concentration is also provided in the Water to Dry Weight ratio or Water/LMB (w/w), and similarly, the alcohol concentration is expressed in the Alcohol to Dry Weight ratio or Alcohol/LMB ratio (w/w).

To an LMBW composition derived from the greatest LMBW zone, more alcohol may be added while maintaining the solution clear and one-phase. However, for safety considerations, the FDA has placed an upper limit for alcohol content at about 20% of the final weight of the formulation for drugs that are injectable directly into blood vessels (e.g. intravenously) or into a soft tissue (e.g. intramuscularly). Therefore, the preferred alcohol concentration in an LMBW is about up to 20% (based on the final weight of the formulation at the time of the injection). Nevertheless, more alcohol can still be added if the formulation is diluted before injection.

It is worthwhile to note that (1) the addition of alcohol is optional for an LMBW composition, and (2) an alcohol-containing LMBW composition will retain all LMBW properties.

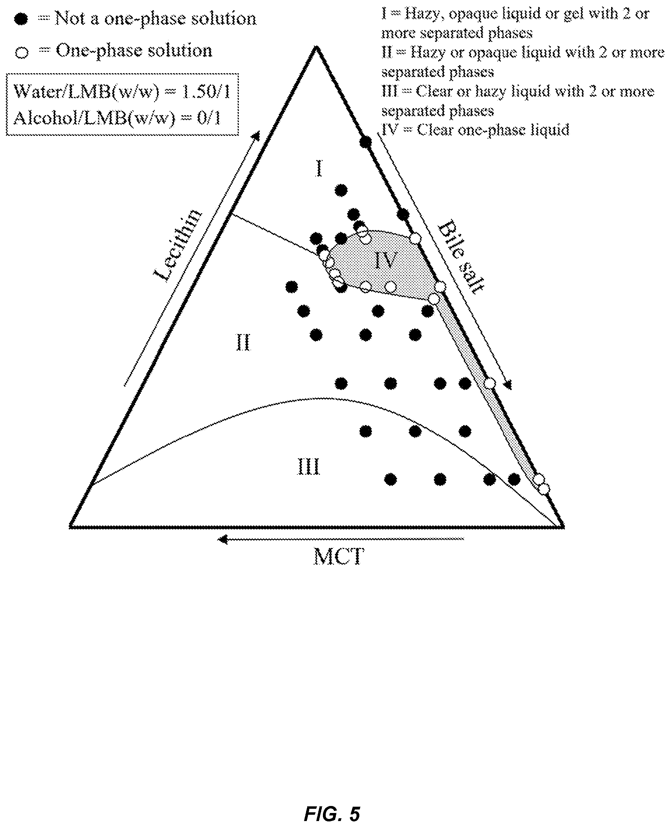

FIG. 5 illustrates a ternary phase diagram for L, M and B without alcohol or Alcohol/LMB (w/w) = 0/1, wherein only within the LMBW zone (Zone IV), a composition is a clear and one-phase solution. In any other region (I, II or III), a composition is not clear or has more than one phases.

It was also observed that any composition of L, M and B outside the LMBW zone would form multi-phase liquids or semi-solids in water, namely emulsions, suspensions, gels, etc. (FIG. 5).

An LMBW can form spontaneously in water or is self-forming. A clear and one-phase composition within an LMBW zone can be easily formed using a low-energy mixer to disperse and dissolve L, M and B in water. No special high temperature, high speed, high pressure, extrusion, microfluidization or high energy homogenization is required. This characteristic of LMBWs contrasts with other lecithin-based compositions such as emulsions, suspensions, or liposomes, all of which require high-energy homogenization methods for their preparation.

Similarly, to dilute an LMBW with water or an IV infusion fluid, only gentle mixing is needed. This readily water-dilute-able feature of an LMBW is particularly useful for IV infusion, wherein the LMBW is provided as a "concentrate" drug formulation and is diluted with water or an IV infusion fluid before administration.

The self-forming nature (no need for high-energy homogenization) of an LMBW also suggests that it is a thermodynamically favorable system and is physically stable. Unlike other lecithin-based formulations, an LMBW will remain as a clear and one-phase solution and will not form precipitates or undergo aggregation or phase separation over time (Example 12). In contrast, an emulsion, liposome, micelle or suspension composition is NOT a thermodynamically favorable system; over time, the components in these systems will aggregate and the system will separate into distinct phases. For example, over time, an emulsion will "cream out" into aggregated and separated phases.

The fact that an LMBW composition is a clear and one-phase solution is highly desirable to the patient, nurse, or physician. The solution transparency allows the user to visually examine the drug preparation before use to ensure there are no solid particles, microbial growth or other aberrant content. This contrasts with most other lecithin-, MCT- or bile salt-based compositions described in the prior art, which are either intrinsically opaque or cloudy (e.g., liposomes, emulsions), or turn milky or precipitate after dilution in water (e.g., solvent-based or micelle solutions).

An LMBW is a ready-to-use formulation that can be injected directly without prior dilution. Such a ready-to-use formulation is desired because it eliminates certain steps of preparation or dilution that can be tedious or prone to medical error. A ready-to-use LMBW formulation is of importance for rescue drugs that require immediate administration under emergency circumstances, and where there is little or no time for dilution.

Within the LMBW zone, various compositions containing different drugs have been selected, prepared and demonstrated to have the desired LMBW properties (see Examples 4-29).

In an aspect, an LMBW may be provided without a drug. A drug is not an essential component of an LMBW. A drug-free LMBW vehicle (Part 1) can be prepared, then subsequently mixed with a drug (Part 2) prior to administration. This two-part system may be desirable for drugs that do not have long-term stability after the two parts are combined.

In an aspect, an LMBW may be provided with a drug in it. To incorporate a drug into an LMBW vehicle, the drug substance is added to an LMBW and then mixed until the drug is dissolved. The drug substance may be added to and dissolved in a pre-made LMBW vehicle, or it may be added at any step during the preparation of an LMBW (Examples 2, 3, 4 and 15).

The LMBW composition is a one-phase liquid and is free of any oil droplets, solid particles, aggregates or super molecular structure that are detectable with the naked eye or by using certain particle-detecting analytical instruments such as a microscope or a DLS spectrometer (Example 13 & Example 16). A one-phase composition such as an LMBW is preferred over multi-phase compositions because of the LMBW's transparency, physical stability, and simple manufacturing process.

The LMBW composition is a one-phase liquid and is free of DLS detectable oil droplets, solid particles, aggregates or super molecular structure of size greater than 5 nm, preferably greater than 2 nm or more preferably greater than 1 nm in diameter.

In an aspect, an LMBW composition is clear with T800 greater than 70%, preferably greater than 80% or more preferably 90%.

In another aspect, an LMBW has a viscosity at between about 1 and 10,000 centipoises or cP, preferably, 10-1,000 cP or more preferably 10-100 cP.

In an aspect, an LMBW can be passed through a 0.2-micron filter membrane. This allows the sterilization of the LMBD composition by simple filtration (Example 3).

In an aspect, an LMBW vehicle (without drug) can also be sterilized by heatsterilization methods such as autoclaving. If a drug is not heat sensitive, then the LMBW containing the drug may also be suitable for heat sterilization.

In an aspect, an LMBW further contains an excipient such as an acid, antioxidant, base, pH buffer, bulking agent, metal chelator, preservative, viscosity enhancer, osmotic pressure adjuster, or inert gas e.g., nitrogen etc.

All components in an LMBW are regarded safe for injection or injectable. The key components L, M and B have been previously approved by the FDA for use in injectable drugs. Phosphatidylcholines, bile salts and triglycerides are endogenous to the human body as they are found in the human blood. Experimentally, LMBW compositions have been shown to be safe in both animals and humans following injection into soft tissue (via subcutaneous injections) and into blood vessels (via intravenous injections) (Examples, 5, 6 & 15).

An LMBW containing an insoluble psychoactive drug diazepam was tested in human for safety and pharmacokinetic profiles following a subcutaneous injection. This was a Phase 1 trial under an IND approved by the FDA. The LMBW formulation was found to be well-tolerated in human subjects without any significant adverse event observed. The same formulation was also tested in rodents and dogs and was found to be safe following subcutaneous injection (Example 15). In an aspect, an LMBW composition containing diazepam is administered by intravenous, intramuscular, subcutaneous injection, by oral ingestion, and/or rectal insertion. In an aspect, an LMBW composition containing diazepam is used for anxiety, alcohol withdrawal syndrome, benzodiazepine withdrawal syndrome, muscle spasms, seizures, trouble sleeping, restless legs syndrome or in emergency situations to stop cluster seizures. In an aspect, an LMBW composition contains 0.1- 3 mg/mL diazepam such as 0.1, 0.5, 1, 1.5, 1.6, 1.7, 1.8, 1.9, 2, 2.1, 2.2, 2,4, 2.5 and/or 3 mg/mL. In an aspect, an LMBW composition contains a diazepam dose of 1-20 mg such as 1, 2, 3, 4, 5, 6, 7, 8, 9, 10, 11, 12, 13, 14, 15, 16, 17, 18, 19, 20 mg. In an aspect, an LMBW composition containing a diazepam is dosed at 0.1-5 mL such as 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.5, 3, 4, or 5 mL. In an aspect, an LMBW composition containing diazepam is dosed at the onset of a seizure, or at a frequency of every 1-24 hours.

Surprisingly, an LMBW composition appeared to have reduced the hemolytic or irritation potential of the bile salt it contains (Example 17). Bile salts are known to cause hemolysis and tissue irritation (Cell Mol Biol Lett. 2001;6(4):881-95), and such toxicities are commonly manifested as injection-site reactions. For example, BELKYRA^{™} injection, which contains the bile salt deoxycholic acid as the active ingredient, is associated with severe injection-site reaction. The package inserts for BELKYRA^{™} injection reveals that the drug causes injection-site bleeding/hematoma in 61% of patients administered the drug subcutaneously. However, such injection-site toxicity was not observed in either animals or humans for an LMBW following subcutaneous injections (Example 15), indicating that the combination of bile salt with M and L in an LMBW composition apparently has reduced or eliminated the hemolysis/tissue irritation problem associated with the bile salt.

An LMBW composition is unique because (1) it can only be made with L, M, and B within the LMBW zone, and (2) none of these 3 components can be readily substituted with other ingredients of similar structure or physical properties. For example, lecithin is a lipophilic surfactant, but other lipophilic surfactants such as Span 40 cannot replace lecithin to form an LMBW. Even different lecithin behaved differently in an LMBW, the preferred lecithin for an LMBW is soy lecithin having phosphatidylcholine at 75% or greater based on the lecithin weight (Example 7). Similarly, the M in an LMBW cannot be substituted with a longer chain triglyceride (LCT) such as sesame oil (Example 8), and the B in an LMBW cannot be substituted by another water-soluble surfactant such as the sodium salt of fatty acids.

To further demonstrate the uniqueness and utility of LMBW compositions, the inventors have prepared and tested numerous other possible binary (two components) or ternary (three component) combinations using the same L, M, B, alcohol and water components and found that none of the binary or ternary compositions had the same LMBW properties (Example 2). Any of these non-LMBW compositions would have one or more of the following drawbacks, including:
a. Not clear or transparent;
b. Not a one-phase solution;
c. Becomes cloudy or precipitates when diluted with water;
d. Requires high shear or high energy homogenization to form;
e. Does not dissolve an insoluble drug to the same extent as an LMBW; and
f. Is hemolytic or irritating to tissue at the injection site (Example 17).

Surprisingly, the components (L, M, B) synergistically provide much greater solubility for an insoluble drug (e.g., voriconazole) when they are together in an LMBW composition than singly or in combinations other than an LMBW (Example 2).

In an aspect, for the same amount of lecithin in the formulation, an LMBW can dissolve a greater amount of an insoluble drug compared to other lecithin-based solubilizing formulations such as liposomes, emulsions, alcohol solutions or mixed micelles.

In an aspect, for the same amount of MCT in the formulation, an LMBW can dissolve a greater amount of an insoluble drug than other MCT-based solubilizing formulations such as emulsions.

In an aspect, for the same amount of bile salt in the formulation, an LMBW can dissolve a greater amount of an insoluble drug than other bile salt-based solubilizing formulations such as micelles or mixed micelle solutions.

In an aspect, for the same amount of alcohol in the formulation, an LMBW can dissolve a greater amount of an insoluble drug than other commonly used alcohol-based solubilizing formulations such as alcohol-water cosolvent.

In an aspect, L, M, B, and W when combined in an LMBW formulation provide solubility enhancement for an insoluble drug that is usually much greater than the sum of solubilities achieved by each component separately, and greater than when the components are combined in a form of liposomes, emulsions, micelles, suspensions, mixed micelles or alcohol solutions (Example 2). In an aspect, L, M and B in an LMBW can synergistically enhance solubility of an insoluble drug in water.

An LMBW can dissolve a soluble drug to obtain a clear solution formulation. Because of the presence of L, M and B, an LMBW may act as a fast- or slow-releasing formulation once injected into a soft tissue.

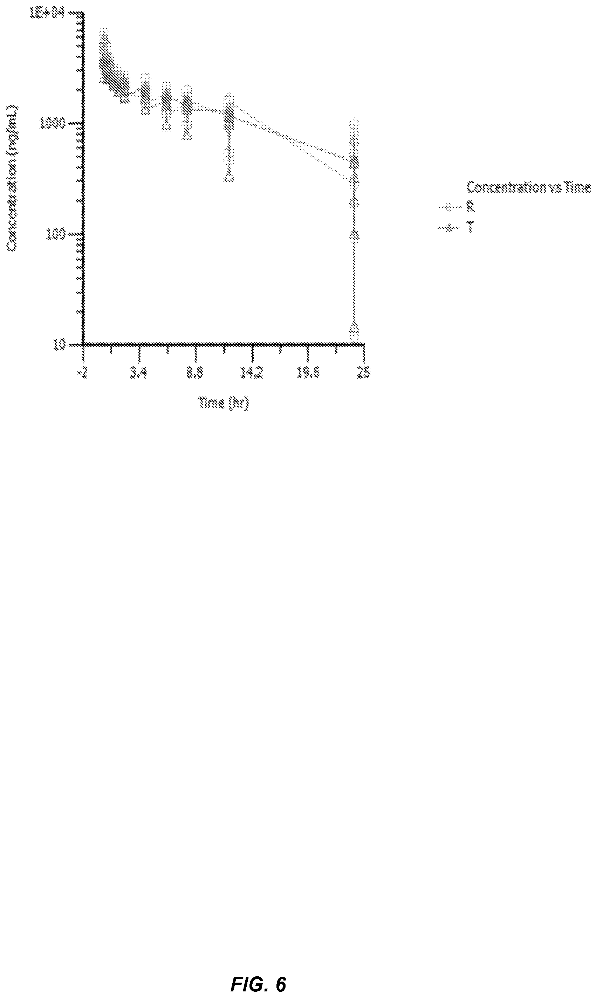

Also, an LMBW compares favorably to many other known drug solubilizers. For example, an LMBW can dissolve the highly insoluble drug voriconazole to the same drug concentration as in the marketed drug product ("VFEND^{®} I.V.") which uses a cyclodextrin as a solubilizer to dissolve voriconazole. Surprisingly, the LMBW formulation for voriconazole exhibited the same pharmacokinetic profiles (FIG. 6) as the VFEND^{®} I.V. formulation yet is free of the kidney toxicity commonly associated with cyclodextrin-based formulations (Example 6). In an aspect, an LMBW composition containing voriconazole is for use in administration by intravenous, intramuscular, subcutaneous injection, oral ingestion, installation into the eyes or ears, and/or rectal insertion. In an aspect, an LMBW composition containing voriconazole for use as an antifungal medication used to treat a number of fungal infections, including but not limited to aspergillosis, candidiasis, coccidioidomycosis, histoplasmosis, penicilliosis, and infections by Scedosporium or Fusarium is provided. In an aspect, an LMBW composition contains 1 - 60 mg/mL voriconazole such as 1, 2, 3, 4, 5, 6, 7, 8, 9, 10, 11, 12, 13, 14, 15, 16, 17, 18, 19, 20, 25, 30, 35, 40, 45, 50, 55, or 60 mg/mL. In an aspect, an LMBW composition contains a voriconazole in a drug to Dry weight ratio between 0.027/1 and 0.16/1. In an aspect, an LMBW composition contains a voriconazole dose of 1-200 mg such as 1, 2, 3, 4, 5, 6, 7, 8, 9, 10, 11, 12, 13, 14, 15, 16, 17, 18, 19, 20, 50, 100, or 200 mg. In an aspect, an LMBW composition containing a voriconazole is dosed at 0.1-20 mL such as 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 3, 4, 5, 10, or 20 mL. In another aspect, an LMBW composition containing voriconazole is lyophilized or substantially free of water. In another aspect, an LMBW composition containing voriconazole can be dosed at a frequency of every 6-24 hours. In yet another aspect, an LMBW composition containing voriconazole is bioequivalent to the VFEND^{®} I.V. formulation.

In an aspect, a drug in an LMBW formulation is safer than a cyclodextrin-based formulation of the same drug.

In an aspect, an LMBW is safe for repeated daily intravenous injection into beagle dogs at 4-6 mL per day.

In an aspect, an LMBW is pharmacokinetically equivalent to or bioequivalent to a cyclodextrin formulation containing the same drug.

In an aspect, an LMBW can provide a greater solubility for an insoluble drug than a cyclodextrin formulation (Example 25).

In an aspect, an LMBW can substitute the cyclodextrin as a solubilizer for the drug products that are currently formulated with cyclodextrins. The cyclodextrin-solubilized drugs include but are not limited to cannabidiol, PGE2, PGE1, opalmon, piroxicam, benexate HCl, iodine, dexamethasone, nitroglycerin, cefotiam-hexetil, cephalosporin, tiaprofenic acid, diphenhydramin and chlortheophyllin, chlordiazepoxide, hydrocortisone, itraconazole, cisapride, nimesulide, alprostadil, nicotine, chloramphenicol, diclofenac sodium, estradiol, indomethacin, omeprazol, posaconazole, tetrahydrocannabinol, voriconazole, ziprasidone mesylate, dextromethorphan, cetirzine, mitomycin, meloxicam, and aripiprazole.

In an aspect, an LMBW can dissolve each of the following insoluble drugs, diagnostic agents or nutritional supplements to a level that is significantly higher than its intrinsic solubility in water: amiodarone, amphotericin, aprepitant, bacitracin, camptothecin, casein, clarithromycin, cyclosporine, diazepam, docetaxel, ibuprofen, celecoxib, itraconazole, irinotecan, iodine, levodopa, paclitaxel, phytonadione, posaconazole, prednisolone acetate, progesterone, propofol, resveratrol, tyrosine, oriconazole, or a salt or a combination thereof (Example 4).

In Example 4, an LMBW composition was shown to increase solubility of drug molecules over a wide range of molecular weights, chemical structures, physicochemical properties and therapeutic uses. Based on these observations, it is reasonable to regard an LMBW composition as a "Universal Solubilizer" for its broad utility in solubilizing insoluble drugs.

In an aspect, an LMBW is substantially-free of drug i.e., the LMBW contains 0% drug. In other aspects, the LMBW composition contains 0-25% w/w drug such as 0, 1, 2, 3, 4, 5, 6, 7, 8, 9, 10, 11, 12, 13, 14, 15, 16, 17, 18, 19, 20, 21, 22, 23, 24, and/or 25% w/w drug. In certain aspects, the amount of drug or active agent is about 0.1 % to about 5%, or about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, and/or 5 % w/w, all based on the final weight of the composition. In other aspects, the LMBW composition contains a drug at 0/1 - 0.5/1 in drug weight ratio to the Dry weight, such as 0/1, 0.1/1, 0.2/1, 0.3/1, 0.4/1, 0.5/1, 0.6/1, 0.7/1, 0.8/1, 0.9/1 and/or 1/1. In another aspects, a LMBW composition contains a drug dose ranging from 0.001 mg to 100 g. In another aspects, a drug-containing LMBW composition is provided in a volume ranging from 0.001 mL to 10 L. In yet another aspect, a drug-containing LMBW composition can be dosed continuously, hourly, 4 times a day, 3 times a day, twice a day, once-a-day, once every 2 days, once every 3 days, once every 4 days, once every 5 days, once every 6 days, once-a-week, once-every 2 weeks, and/or monthly.

The drug solubilization potential of a cyclodextrin is limited by a drug's ability to form an inclusion complex with the cyclodextrin. The inclusion complex requires the drug molecule to have certain molecular weight, size and structure. In other words, unlike an LMBW, cyclodextrin cannot be considered a Universal Solubilizer.

In another aspect, an LMBW can also be used to dissolve and/or deliver a diagnostic agent or nutritional supplement. An LMBW for such purposes can be administered to a human or animal patient by injection or instillation, or by oral, topical, ophthalmic, otic, nasal, inhalation, vaginal, or rectal administration.

In one aspect, an LMBW vehicle alone that does not contain a drug could also have medical utility. For example, an LMBW may be injected into fat tissue to dissolve fats for therapeutic or cosmetic purposes like BELKYRA^{™} (Example 29). In addition, an LMBW solution may be used as a biocompatible cleansing liquid to clean body parts such as skin, eye, ear, nose, vagina, rectum, wound (debridement with or without enzymes), mucus, soft tissue, or hair, etc., for therapeutic, personal care or cosmetic purposes.

In an aspect, an LMBW composition can be used to dissolve and remove undesirable insoluble solid deposits in human or animal bodies such as uric acid deposits as in gout, kidney stones, cholesterol deposits in atherosclerosis or xanthelasma and the protein deposits in Alzheimer's (amyloids) (Example 29).

In an aspect, an LMBW can be injected as is or is ready-to-use. In another aspect, an LMBW is a concentrate, in yet another aspect, an LMBW is both a ready-to-use and a concentrate formulation. and in yet another aspect, an LMBW is initially provided in an anhydrous form and formed upon mixing with water.

The present invention provides a clear and one-phase aqueous solution composition as claimed, comprising (a) lecithin at a concentration between 6% and 72.5%, (b) MCT at a concentration between >0% and 32.5% and (c) a bile salt at a concentration between 11.5% and 94%, wherein all concentrations are in weight percentage over the Dry Weight and the solution composition further contains alcohol at a concentration of 0.49/1 or less in Alcohol to Dry Weight Ratio.

In an aspect, the present invention provides a clear and one-phase aqueous solution composition of the invention, comprising (a) lecithin at a concentration between about 8% and about 61.5%, (b) MCT at a concentration between about 0% and about 23% and (c) a bile salt at a concentration between about 22% and about 90%, wherein all concentrations are in weight percentage over the Dry Weight and the solution composition is substantially free of alcohol.

A clear and one-phase aqueous solution composition, comprising lecithin, MCT and bile salt in a composition forms within an LMBW zone in a ternary phase diagram having lecithin, MCT and bile salt as the three components, wherein the LMBW zone is enclosed by the following Border points:

| Labels of Border points as in FIG. 1 | Concentration (in Weight Percent over the Dry Weight) | | |
|---|---|---|---|
| | Lecithin | MCT | Bile salt |
| A | 72.5 | 0 | 27.5 |
| B | 61 | 27 | 12 |
| C | 56 | 32.5 | 11.5 |
| D | 52 | 32.5 | 15.5 |
| E | 50 | 10 | 40 |
| F | 6 | 0 | 94 |

Furthermore, the solution composition contains alcohol at a concentration of 0.49/1 or less in Alcohol to Dry Weight Ratio.

A clear and one-phase aqueous solution composition, comprising lecithin, MCT and bile salt in a composition forms within an LMBW zone in a ternary phase diagram having lecithin, MCT and bile salt as the three components, wherein the LMBW zone is defined by connecting the following Border points in a circle:

| Labels of Border points as in FIG. 4 | Concentration in Weight Percent over the Dry weight) | | |
|---|---|---|---|
| | Lecithin | MCT | Bile salt |
| A | 60 | 0 | 40 |
| B | 61.5 | 10 | 28.5 |
| C | 55 | 23 | 22 |
| D | 51 | 23 | 26 |
| E | 50 | 10 | 40 |
| F | 8 | 0 | 92 |

Furthermore, the solution composition is substantially free of alcohol ("alcoholfree").

In another aspect, an LMBW composition of this invention contains alcohol at a concentration between 0/1 and 0.49/1 in Alcohol to Dry Weight Ratio. At 0/1 Alcohol to Dry Weight ratio, the smallest LMBW zone was obtained (FIG. 4) and at 0.49/10/1 Alcohol to Dry Weight ratio, the largest LMBW zone was obtained (FIG. 1).

These LMBW zones are very useful in drug formulation especially for selection of a formulation vehicle to solubilize an insoluble drug. For a drug formulation that could contain some alcohol, one could select several (3 or 4) LMBW compositions at different positions within the largest LMBW zone (FIG. 1) as the formulation vehicles, then measure and compare the solubility of the insoluble drug achieved by these LMBW vehicles and select one preferred vehicle that provided the greatest solubility.

In another aspect, an LMBW composition of this invention contains alcohol at a concentration no more than 1.6/1 in Alcohol to Dry Weight Ratio. A higher alcohol concentration leads to precipitation (Example 18).

In another aspect, an LMBW composition of this invention contains alcohol at a concentration no more than 10%, 20%, 30%, 40%, 50%, 60%, or 70% of the total weight of the final formulation.

In another aspect, alcohol is selected from the group consisting of ethanol, benzyl alcohol, propylene glycol, polyethylene glycol of a low molecular weight (100-5,000 g/mol) or a combination thereof. The preferred alcohol for an LMBW is ethanol, benzyl alcohol, propylene glycol or a combination thereof.

For a drug formulation in which an alcohol is not desired, a similar selection process may be applied using the LMBW zone in FIG. 4, where one could select several (3 or 4) LMBW compositions at different positions within the LMBW zone as the formulation vehicles, then measure and compare the solubility of the insoluble drug achieved by these vehicles, and select one preferred vehicle that provided the greatest solubility.

In another aspect, an LMBW composition of this invention has a pH between about 3 and about 10. In a preferred aspect, the composition of this invention has a pH between about 4 and about 9. In a more preferred aspect, the composition of this invention has a pH between about 4 and about 8. In another aspect, an LMBW composition of this invention has a pH at 3.3, 3.4, 3.5, 3.6, 3.7, 3.8, 3.9, 4, 4.1, 4,2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.0, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8.8, 8.1, 8.2, 8.3, 8.4, 8.5, 8.6, 8.7, 8.8, 8.9, or 9.0.

In another aspect, the composition of an LMBW has an acid and/or base for pH adjustment. The preferred acid is hydrochloric acid and the preferred base is sodium hydroxide.

In yet another aspect, an LMBW contains a preservative. The preferred preservative is benzalkonium chloride, cresol, metacresol (m-cresol), phenol, parabens, benzyl alcohol, EDTA or a mixture thereof. The concentrations that may be used are about 0.01% to 1% for benzalkonium chloride, about 0.08 to 0.315% for cresol/metacresol, 0.06 to 1.3% for phenol, about 0.01 to 1.5% for a paraben, about 0.05 to 10% for benzyl alcohol, about 0.005 to 0.2% for EDTA disodium, and about 0.005 to 0.34% for EDTA calcium disodium. All percentages are on a weight of the individual component to the weight of the composition basis.

In one aspect, An LMBW further contains an antioxidant. The useful antioxidants may include but are not limited to an inert gas, methionine, cysteine, dextrose, fructose, lactose, and a metal chelator such as a salt of edetate (EDTA), or combination thereof. A preferred antioxidant is a combination of methionine and EDTA. The concentration of each antioxidant may be determined based on its stabilizing effect on any component in the composition of this invention and its safety to the patient. A normal range of concentration for each antioxidant can be found in the FDA's Inactive Ingredient List. For example, the methionine concentration range useful for injectable formulations is 0.01% to 49.2% by weight of the composition.

In yet another aspect, an LMBW comprises a bulking agent. The preferred bulking agent is mannitol, sucrose, lactose, trehalose, maltose, starch or a mixture thereof. The concentrations may be used are about 1% to 20% by weight of the composition.

In another aspect, an LMBW provides a method to prepare a drug-free LMBW composition, comprising (a) dissolving a lecithin to a concentration between 6% and 72.5%, a medium chain triglyceride (MCT) to a concentration between >0% and 32.5% and a bile salt to a concentration between 11.5% and 94%, all in weight percent over the total weight of lecithin, MCT and bile salt, in water to a concentration no less than 0.1/1 (w/w) in water to the total weight of lecithin, MCT and bile salt ratio, to form a clear one-phase aqueous solution composition, and (b) adjusting pH to between 3 and 10.

In another aspect, the present invention provides a method to prepare a drug-containing LMBW composition, comprising: comprising (a) dissolving a lecithin to a concentration between 6% and 72.5%, a medium chain triglyceride (MCT) to a concentration between >0% and 32.5% and a bile salt to a concentration between 11.5% and 94%, all in weight percent over the total weight of lecithin, MCT and bile salt, in water to a concentration no less than 0.1/1 (w/w) in water to the total weight of lecithin, MCT and bile salt ratio, to form a clear one-phase aqueous solution composition, and (b) adjusting pH to between 3 and 10.where the drug can be added to LMBW composition and mixed to dissolve at any step.

In another aspect, the present invention provides a method to prepare a drug-containing LMBW composition, comprising (a) dissolving a lecithin to a concentration between 6% and 72.5%, a medium chain triglyceride (MCT) to a concentration between >0% and 32.5% and a bile salt to a concentration between 11.5% and 94%, all in weight percent over the total weight of lecithin, MCT and bile salt, in water to a concentration no less than 0.1/1 (w/w) in water to the total weight of lecithin, MCT and bile salt ratio, to form a clear one-phase aqueous solution composition, and (b) adjusting pH to between 3 and 10, where the drug or a pharmaceutical excipient(s) can be added to the LMBW composition and mixed to dissolve at any step. The pharmaceutical excipient(s) may be alcohol, acid, base, pH buffer, antioxidant, bulking agent, viscosity enhancing agent, osmotic adjusting agent, preservative or a combination thereof.

In another aspect, the present invention provides a method to prepare a drug-containing LMBW composition comprising (a) dissolving a lecithin to a concentration between 6% and 72.5%, a medium chain triglyceride (MCT) to a concentration between >0% and 32.5% and a bile salt to a concentration between 11.5% and 94%, all in weight percent over the total weight of lecithin, MCT and bile salt, in water to a concentration no less than 0.1/1 (w/w) in water to the total weight of lecithin, MCT and bile salt ratio, to form a clear one-phase aqueous solution composition, and (b) adjusting pH to between 3 and 10, where the pH of the liquid is adjusted with an acid or base at any step.

In another aspect, the present invention provides a method to prepare a drug-containing LMBW composition, comprising (a) dissolving a lecithin to a concentration between 6% and 72.5%, a medium chain triglyceride (MCT) to a concentration between >0% and 32.5% and a bile salt to a concentration between 11.5% and 94%, all in weight percent over the total weight of lecithin, MCT and bile salt, in water to a concentration no less than 0.1/1 (w/w) in water to the total weight of lecithin, MCT and bile salt ratio, to form a clear one-phase aqueous solution composition, and (b) adjusting pH to between 3 and 10, which is subsequently filtered through a membrane.

The solution formulation of the present invention can be administered as is (undiluted) or diluted with water prior to administration. Dilutions can also be made using IV infusion fluids. The route of administration may include, but is not limited to, injection and inhalation, or by oral, otic, nasal, topical, ophthalmic, vaginal, and rectum administration. The solution formulation of the present invention can be delivered using devices including, but not limited to, needles/syringes, infusion sets, infusion pump, catheters, applicators, bottles, sprayers, inhalation devices, and wound dressing.

In one aspect, the solution composition of this invention is autoclaved.

In one aspect, the solution composition of this invention is stable for 6 months or longer.

In one aspect, the composition of this invention has an osmotic pressure of about 100 to 600 mOsmol/L.

In one aspect, the composition of this invention is for use in delivering a drug into a human or animal patient.

In one aspect, the composition of this invention is for use in treatment, prevention or diagnosis of a disease in a human or in an animal.

In one aspect, the composition of this invention is for use in nutritional supplements formulations.

The present invention will be further understood by reference to the following nonlimiting examples.

## Claims

1. An injectable solubilizer composition, comprising:
a. a lecithin, having phosphatidylcholine content no less than 75% w/w based on the lecithin weight, at a concentration between 6% and 72.5%;
b. a medium chain triglyceride (MCT) having fatty acids of chain length of 6 to 12 carbons, at a concentration between >0% and 32.5%;
c. a bile salt, at a concentration between 11.5% and 90%;
d. water, and
e. optionally alcohol; wherein the % is the weight percentage over the total weight of lecithin, MCT and bile salt and wherein the said composition is a clear and one-phase aqueous solution, and is substantially free of solid particles, or oil droplets or other detectable aggregates or super molecular structures in the size range of >5 nm or greater, and wherein the pH of the composition is between 3 and 10.

2. The composition according to claim **1,** wherein the water concentration is no less than 0.1/1, in weight ratio of water to the total weight of lecithin, MCT and bile salt; and
wherein the composition is substantially free of liposome, emulsion, phospholipid particles or oil droplets of size greater than 5 nanometers in diameter.

3. The composition according to claim **1,** wherein the lecithin or MCT is derived from a natural source or is synthetically made.

4. The composition according to claim **1,** wherein the bile salt is selected from a group consisting of sodium, potassium, lithium, calcium, arginine, lysine or ammonium salts of cholic acid, glycocholic acid, taurocholic acid, deoxycholic acid, glyco- or taurodeoxycholic acid, chenodeoxycholic acid, glyco- or taurochenoxydeoxycholic acid, or a mixture thereof.

5. The composition according to claim **1,** wherein the composition further contains an alcohol selected from a group consisting of ethanol, benzyl alcohol, propylene glycol, polyethylene glycol of a low molecular weight (100-5,000 g/mol) or a combination thereof.

6. The composition according to claim **1,** wherein the composition further contains a soluble or an insoluble drug, wherein the insoluble drug is one or more insoluble drug(s) selected from a group comprising amiodarone, amphotericin, aprepitant, camptothecin, casein, celecoxib, clarithromycin, cyclosporine, diazepam, docetaxel, ibuprofen, iodine, itraconazole, iodine, irinotecan, levodopa, paclitaxel, phytonadione, posaconazole, prednisolone acetate, progesterone, propofol, resveratrol, tetrahydrocannabinol, tyrosine, voriconazole, or salt thereof.

7. The composition according to claim 1, wherein the drug is selected from a group consisting of small molecules, peptides, proteins or a combination thereof.

8. The composition according to claim **1,** further comprising voriconazole at a concentration between 0.01% and 3%, wherein the bile salt is glycocholic acid at a concentration between 11.5% and 94%, wherein the % is the weight percentage over the total weight of lecithin, MCT and glycocholic acid, and pH of the solution is between 3.5 and 7.5.

9. The composition according to claim **1,** further comprising diazepam at a concentration between 0.01% and 3%, wherein the bile salt is glycocholic acid at a concentration between 11.5% and 94%, wherein the % is the weight percentage over the total weight of lecithin, MCT and glycocholic acid, and pH of the solution is between 6 and 8.5.

10. The composition according to claim **1,** wherein the composition further comprises a preservative selected from a group consisting of cresols, phenol, benzyl alcohol, ethanol, chlorobutanol, parabens, imidura, benzylkonium chloride, EDTA or a salt or a combination thereof; or
wherein the composition further comprises a bulking agent selected from a group consisting of mannitol, sorbitol, xylitol, lactose, fructose, xylose, sucrose, trehalose, mannose, maltose, dextrose, dextran, dextrin, or a combination thereof; or
wherein the composition further comprises an antioxidant selected from: EDTA, citric acid, glycine, butylated hydroxyanisole, butylated hydroxytoluene, cysteine, methionine, thioglycerol, bisulfite, metabisulfate, ascorbate, propyl gallate, tocopherol, reducing sugar, or a salt or combination thereof.

11. A method for preparation of a composition according to claim **1,** comprising (a) dissolving a lecithin to a concentration between 6% and 72.5%, a medium chain triglyceride (MCT) to a concentration between >0% and 32.5% and a bile salt to a concentration between 11.5% and 94%, all in weight percent over the total weight of lecithin, MCT and bile salt, in water to a concentration no less than 0.1/1 (w/w) in water to the total weight of lecithin, MCT and bile salt ratio, to form a clear one-phase aqueous solution composition, and (b) adjusting pH to between 3 and 10.

12. The composition according to any one of the preceding claims for use in treatment, prophylaxis, diagnosis, or a nutritional supplement in a human or animal subject.

## Patentansprüche

1. Eine injizierbare Lösungsvermittler-Zusammensetzung, umfassend:
**a.** ein Lecithin mit einem Phosphatidylcholin-Gehalt von nicht weniger als 75 Gew.-%, bezogen auf das Gewicht des Lecithins, in einer Konzentration zwischen 6 % und 72,5 %;
**b.** ein mittelkettiges Triglycerid (MCT) mit Fettsäuren einer Kettenlänge von 6 bis 12 Kohlenstoffatomen in einer Konzentration zwischen >0 % und 32,5 %;
**c.** ein Gallensalz in einer Konzentration zwischen 11,5 % und 90 %;
**d.** Wasser und
**e.** optional Alkohol; wobei der Prozentsatz der Gewichtsprozentsatz bezogen auf das Gesamtgewicht von Lecithin, MCT und Gallensalz ist und wobei die genannte Zusammensetzung eine klare und einphasige wässrige Lösung ist und im Wesentlichen frei von festen Partikeln oder Öltröpfchen oder anderen nachweisbaren Aggregaten oder supramolekularen Strukturen im Größenbereich von >5 nm oder größer ist, und wobei der pH-Wert der Zusammensetzung zwischen 3 und 10 liegt.

2. Die Zusammensetzung gemäß Anspruch **1**, wobei die Wasserkonzentration nicht weniger als 0,1/1 beträgt, im Gewichtsverhältnis von Wasser zum Gesamtgewicht von Lecithin, MCT und Gallensalz; und
wobei die Zusammensetzung im Wesentlichen frei ist von Liposom, Emulsion, Phospholipidpartikeln oder Öltröpfchen mit einer Größe von mehr als 5 Nanometern im Durchmesser.

3. Die Zusammensetzung gemäß Anspruch 1, wobei das Lecithin oder MCT aus einer natürlichen Quelle stammt oder synthetisch hergestellt ist.

4. Die Zusammensetzung gemäß Anspruch 1, wobei das Gallensalz aus einer Gruppe ausgewählt ist, die aus Natrium-, Kalium-, Lithium-, Calcium-, Arginin-, Lysin- oder Ammoniumsalzen von Cholsäure, Glykocholsäure, Taurocholsäure, Desoxycholsäure, Glyko- oder Taurodesoxycholsäure, Chenodesoxycholsäure, Glyko- oder Taurochenoxydeoxycholsäure oder einer Mischung davon besteht.

5. Die Zusammensetzung gemäß Anspruch 1, wobei die Zusammensetzung ferner einen Alkohol enthält, der aus einer Gruppe ausgewählt ist, die aus Ethanol, Benzylalkohol, Propylenglykol, Polyethylenglykol mit niedrigem Molekulargewicht (100-5.000 g/mol) oder einer Kombination davon besteht.

6. Die Zusammensetzung gemäß Anspruch 1, wobei die Zusammensetzung ferner ein lösliches oder ein unlösliches Arzneimittel enthält, wobei das unlösliche Arzneimittel ein oder mehrere unlösliche Arzneimittel ist, ausgewählt aus einer Gruppe, die Amiodaron, Amphotericin, Aprepitant, Camptothecin, Kasein, Celecoxib, Clarithromycin, Ciclosporin, Diazepam, Docetaxel, Ibuprofen, Jod, Itraconazol, Jod, Irinotecan, Levodopa, Paclitaxel, Phytomenadion, Posaconazol, Prednisolonacetat, Progesteron, Propofol, Resveratrol, Tetrahydrocannabinol, Tyrosin, Voriconazol oder ein Salz davon umfasst.

7. Die Zusammensetzung gemäß Anspruch 1, wobei das Arzneimittel aus einer Gruppe ausgewählt ist, die aus kleinen Molekülen, Peptiden, Proteinen oder einer Kombination davon besteht.

8. Die Zusammensetzung gemäß Anspruch 1, die ferner Voriconazol in einer Konzentration zwischen 0,01 % und 3 % umfasst, wobei das Gallensalz Glykocholsäure in einer Konzentration zwischen 11,5 % und 94 % ist, wobei der Prozentsatz der Gewichtsprozentsatz bezogen auf das Gesamtgewicht von Lecithin, MCT und Glykocholsäure ist und der pH-Wert der Lösung zwischen 3,5 und 7,5 liegt.

9. Die Zusammensetzung gemäß Anspruch 1, die ferner Diazepam in einer Konzentration zwischen 0,01 % und 3 % umfasst, wobei das Gallensalz Glykocholsäure in einer Konzentration zwischen 11,5 % und 94 % ist, wobei der Prozentsatz der Gewichtsprozentsatz bezogen auf das Gesamtgewicht von Lecithin, MCT und Glykocholsäure ist und der pH-Wert der Lösung zwischen 6 und 8,5 liegt.

10. Die Zusammensetzung gemäß Anspruch 1, wobei die Zusammensetzung ferner ein Konservierungsmittel umfasst, das aus einer Gruppe ausgewählt ist, die aus Kresolen, Phenol, Benzylalkohol, Ethanol, Chlorbutanol, Parabenen, Imidurea, Benzalkoniumchlorid, EDTA oder einem Salz oder einer Kombination davon besteht; oder
wobei die Zusammensetzung ferner einen Füllstoff umfasst, ausgewählt aus einer Gruppe, die aus Mannitol, Sorbitol, Xylitol, Laktose, Fruktose, Xylose, Saccharose, Trehalose, Mannose, Maltose, Dextrose, Dextran, Dextrin oder einer Kombination davon besteht; oder
wobei die Zusammensetzung ferner ein Antioxidans umfasst, ausgewählt aus: EDTA, Zitronensäure, Glycin, Butylhydroxyanisol, Butylhydroxytoluol, Cystein, Methionin, Thioglycerin, Bisulfit, Metabisulfit, Ascorbat, Propylgallat, Tocopherol, reduzierendem Zucker oder einem Salz oder einer Kombination davon.

11. Ein Verfahren zur Herstellung einer Zusammensetzung gemäß Anspruch 1, umfassend (a) Auflösen von Lecithin in einer Konzentration zwischen 6 % und 72,5 %, eines mittelkettigen Triglycerids (MCT) in einer Konzentration zwischen >0 % und 32,5 % und eines Gallensalzes in einer Konzentration zwischen 11,5 % und 94 %, jeweils in Gewichtsprozent bezogen auf das Gesamtgewicht von Lecithin, MCT und Gallensalz, in Wasser auf eine Konzentration von nicht weniger als 0,1/1 (Gew./Gew.) in Wasser, bezogen auf das Gesamtgewicht von Lecithin, MCT und Gallensalz, um eine klare, einphasige wässrige Lösungszusammensetzung zu bilden, und (b) Einstellen des pH-Werts auf einen Wert zwischen 3 und 10.

12. Die Zusammensetzung gemäß einem der vorstehenden Ansprüche zur Verwendung bei der Behandlung, Prophylaxe, Diagnose oder als Nahrungsergänzungsmittel bei einem menschlichen oder tierischen Patienten.

## Revendications

1. Composition solubilisante pour injection, comprenant :
a. une lécithine, ayant une teneur en phosphatidylcholine qui n'est pas inférieure à 75 % en poids par rapport au poids de la lécithine, à une concentration comprise entre 6 % et 72,5 % ;
b. un triglycéride à chaîne moyenne (MCT) ayant des acides gras d'une longueur de chaîne de 6 à 12 carbones, à une concentration comprise entre > 0 % et 32,5 % ;
c. un sel biliaire, à une concentration comprise entre 11,5 % et 90 % ;
d. de l'eau, et
e. éventuellement de l'alcool ; dans laquelle le % est le pourcentage en poids par rapport au poids total de la lécithine, du MCT et du sel biliaire et dans laquelle ladite composition est une solution aqueuse transparente et monophasique, et est essentiellement exempte de particules solides, ou de gouttelettes d'huile ou d'autres agrégats détectables ou de superstructures moléculaires dans la plage de taille de > 5 nm ou plus, et dans laquelle le pH de la composition est compris entre 3 et 10.

2. Composition selon la revendication 1, dans laquelle la concentration en eau n'est pas inférieure à 0,1/1, en rapport pondéral de l'eau au poids total de la lécithine, du MCT et du sel biliaire ; et
dans laquelle la composition est essentiellement exempte de liposome, d'émulsion, de particules de phospholipide ou de gouttelettes d'huile d'une taille supérieure à 5 nanomètres de diamètre.

3. Composition selon la revendication 1, dans laquelle la lécithine ou le MCT est dérivé d'une source naturelle ou est fabriqué de manière synthétique.

4. Composition selon la revendication 1, dans laquelle le sel biliaire est choisi dans un groupe constitué par les sels de sodium, de potassium, de lithium, de calcium, d'arginine, de lysine ou d'ammonium d'acide cholique, d'acide glycocholique, d'acide taurocholique, d'acide désoxycholique, d'acide glyco- ou taurodésoxycholique, d'acide chénodésoxycholique, d'acide glyco- ou taurochénoxydésoxycholique, ou d'un mélange de ceux-ci.

5. Composition selon la revendication 1, dans laquelle la composition contient en outre un alcool choisi dans un groupe constitué par l'éthanol, l'alcool benzylique, le propylène glycol, le polyéthylène glycol de masse moléculaire faible (100 à 5000 g/mol) ou une combinaison de ceux-ci.

6. Composition selon la revendication 1, dans laquelle la composition contient en outre un médicament soluble ou insoluble, dans laquelle le médicament insoluble est un ou plusieurs médicaments insolubles choisis dans un groupe comprenant l'amiodarone, l'amphotéricine, l'aprépitant, la camptothécine, la caséine, le célécoxib, la clarithromycine, la cyclosporine, le diazépam, le docétaxel, l'ibuprofène, l'iode, l'itraconazole, l'iode, l'irinotécan, la lévodopa, le paclitaxel, la phytonadione, le posaconazole, la prednisolone acétate, la progestérone, le propofol, le resvératrol, le tétrahydrocannabinol, la tyrosine, le voriconazole, ou un sel de ceux-ci.

7. Composition selon la revendication 1, dans laquelle le médicament est choisi dans un groupe constitué par les petites molécules, les peptides, les protéines ou une combinaison de ceux-ci.

8. Composition selon la revendication 1, comprenant en outre du voriconazole à une concentration comprise entre 0,01 % et 3 %, dans laquelle le sel biliaire est l'acide glycocholique à une concentration comprise entre 11,5 % et 94 %, dans laquelle le % est le pourcentage en poids par rapport au poids total de la lécithine, du MCT et de l'acide glycocholique, et le pH de la solution est compris entre 3,5 et 7,5.

9. Composition selon la revendication 1, comprenant en outre du diazépam à une concentration comprise entre 0,01 % et 3 %, dans laquelle le sel biliaire est l'acide glycocholique à une concentration comprise entre 11,5 % et 94 %, dans laquelle le % est le pourcentage en poids par rapport au poids total de la lécithine, du MCT et de l'acide glycocholique, et le pH de la solution est compris entre 6 et 8,5.

10. Composition selon la revendication 1, dans laquelle la composition comprend en outre un conservateur choisi dans un groupe constitué par les crésols, le phénol, l'alcool benzylique, l'éthanol, le chlorobutanol, les parabènes, l'imidurea, le chlorure de benzalkonium, l'EDTA ou un sel ou une combinaison de ceux-ci ; ou
dans laquelle la composition comprend en outre un agent gonflant choisi dans un groupe constitué par le mannitol, le sorbitol, le xylitol, le lactose, le fructose, le xylose, le sucrose, le tréhalose, le mannose, le maltose, le dextrose, le dextrane, la dextrine, ou une combinaison de ceux-ci ; ou
dans laquelle la composition comprend en outre un antioxydant choisi parmi : l'EDTA, l'acide citrique, la glycine, l'hydroxyanisole butylé, l'hydroxytoluène butylé, la cystéine, la méthionine, le thioglycérol, le bisulfite, le métabisulfite, l'ascorbate, le gallate de propyle, le tocophérol, un sucre réducteur, ou un sel ou une combinaison de ceux-ci.

11. Procédé de préparation d'une composition selon la revendication 1, comprenant les étapes consistant à (a) dissoudre une lécithine jusqu'à une concentration comprise entre 6 % et 72,5 %, un triglycéride à chaîne moyenne (MCT) jusqu'à une concentration comprise entre > 0 % et 32,5 % et un sel biliaire jusqu'à une concentration comprise entre 11,5 % et 94 %, tous en pourcentage en poids par rapport au poids total de la lécithine, du MCT et du sel biliaire, dans l'eau jusqu'à une concentration qui n'est pas inférieur à 0,1/1 en poids dans l'eau par rapport au rapport en poids total de la lécithine, du MCT et du sel biliaire, pour former une composition de solution aqueuse transparente monophasique, et (b) ajuster le pH jusqu'à entre 3 et 10.

12. Composition selon l'une quelconque des revendications précédentes pour une utilisation dans le traitement, la prophylaxie, le diagnostic, ou un supplément nutritionnel chez un sujet humain ou animal.
